(19) Europäisches Patentamt
European Patent Office
Office européen des brevets

(11) EP 2 962 703 B1

(12) EUROPEAN PATENT SPECIFICATION

(45) Date of publication and mention of the grant of the patent:
**19.08.2020 Bulletin 2020/34**

(21) Application number: **14757194.7**

(22) Date of filing: **27.02.2014**

(51) Int Cl.:
***A61L 27/22*** *(2006.01)* ***A61L 27/38*** *(2006.01)*
***A61L 27/56*** *(2006.01)* ***A61L 27/58*** *(2006.01)*
***C12N 5/00*** *(2006.01)*

(86) International application number:
**PCT/JP2014/054882**

(87) International publication number:
**WO 2014/133081 (04.09.2014 Gazette 2014/36)**

# (54) CELL CONSTRUCT FOR CELL TRANSPLANTATION, BIOCOMPATIBLE POLYMER BLOCK, AND METHOD FOR PRODUCING THE SAME

ZELLKONSTRUKT FÜR ZELLTRANSPLANTATION, BIOKOMPATIBLER POLYMERBLOCK UND VERFAHREN ZUR HERSTELLUNG DAVON

STRUCTURE CELLULAIRE POUR TRANSPLANTATION CELLULAIRE, BLOC DE POLYMÈRE BIOCOMPATIBLE ET PROCÉDÉS POUR LES PRODUIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2013 JP 2013036942**

(43) Date of publication of application:
**06.01.2016 Bulletin 2016/01**

(73) Proprietor: **FUJIFILM Corporation**
**Tokyo 106-8620 (JP)**

(72) Inventors:
- **NAKAMURA, Kentaro**
**Ashigarakami-gun**
**Kanagawa 258-8577 (JP)**
- **IWAZAWA, Reiko**
**Ashigarakami-gun**
**Kanagawa 258-8577 (JP)**
- **MIYOSHI, Hayato**
**Ashigarakami-gun**
**Kanagawa 258-8577 (JP)**
- **YAMAGUCHI, Kazuhiro**
**Ashigarakami-gun**
**Kanagawa 258-8577 (JP)**
- **FUSHIMI, Hideo**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 543 397**
**EP-A1- 2 543 398**
**WO-A1-2011/108517**
**JP-A- S6 226 230**
**JP-A- 2010 083 788**
**JP-A- 2012 219 100**
**JP-B1- 4 422 191**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 962 703 B1

## Description

### Technical Field

The present invention relates to a cell construct for cell transplantation, a biocompatible polymer block, and a method for producing the same. More specifically, the present invention relates to a cell construct for cell transplantation, in which the necrosis of cells after the transplantation is suppressed, a biocompatible polymer block used in the production of such a cell construct for cell transplantation, and a method for producing the same.

### Background Art

The practical utilization of regenerative medicine, which helps regeneration of living tissues/organs that have fallen into functional disorder or functional incompetence, is currently proceeding. The regenerative medicine is a novel medical technology of recreating the same or similar forms or functions as in original tissues using 3 factors, i.e., cells, scaffolds, and growth factors, for living tissues that could be no longer recovered by only natural healing ability intrinsically possessed by organisms. In recent years, treatments using cells have been being gradually realized. Examples of such treatments include cultured epidermis using autologous cells, cartilage treatment using autologous cartilage cells, bone regeneration treatment using mesenchymal stem cells, cardiac muscle cell sheet treatment using myoblasts, corneal regeneration treatment using corneal epithelial sheets, and nerve regeneration treatment. These novel treatments, unlike conventional alternative medicine based on artificial materials (e.g., bone prosthetic materials, hyaluronic acid injection, etc.), are directed towards repairing and/or regenerating living tissues, and therefore produce high therapeutic effects. In fact, some products such as cultured epidermis or cultured cartilage using autologous cells have been commercially available.

In this context, upon regeneration of cardiac muscle using cell sheets for example, it is considered that regeneration of thick tissues requires a multilayer construct of cell sheets. In recent years, Okano et al. have developed cell sheets using a temperature-responsive culture dish. The cell sheets do not require treatment with an enzyme such as trypsin, and thus retain cell-to-cell binding and adhesion proteins (Non Patent Literatures 1 to 6). Such a cell sheet production technique is expected to be useful in the regeneration of cardiac muscle tissues (Non Patent Literature 7). Moreover, Okano et al. are developing cell sheets also containing vascular endothelial cells introduced therein, in order to form vascular network in the cell sheets (Non Patent Literature 8).

Furthermore, Patent Literature 1 describes a three-dimensional cell construct produced by three-dimensionally arranging polymer blocks having biocompatibility and cells in a mosaic pattern. In the case of this three-dimensional cell construct, it is possible to deliver nutrient from the outside into the three-dimensional cell construct, and further, the three-dimensional cell construct has a sufficient thickness and cells are uniformly present in the construct. Further, in the Examples of Patent Literature 1, high cell survival activity has been demonstrated using a polymer block composed of recombinant gelatin or a natural gelatin material.

EP 2 543 397 describes a cell construct comprising polymer blocks, preferably of recombinant gelatin, and cells, wherein the polymer blocks are arranged in spaces between the cells and have a size from 10-300 μm. The polymer blocks may be cross-linked, preferably by glutaraldehyde or by thermal treatment. The polymer blocks are made by freezing a solution of the polymer followed by freeze-drying.

### Prior Art Literatures

#### Patent Literatures

Patent Literature 1: International Publication No. WO2011/108517

#### Non Patent Literatures

Non Patent Literature 1: Shimizu, T. et al., Circ. Res. 90, e40-48 (2002)
Non Patent Literature 2: Kushida, A. et al., J. Biomed. Mater. Res. 51, 216-223 (2000)
Non Patent Literature 3: Kushida, A. et al., J. Biomed. Mater. Res. 45, 355-362 (1999)
Non Patent Literature 4: Shimizu, T., Yamato, M., Kikuchi, A. & Okano, T., Tissue Eng. 7, 141-151 (2001)
Non Patent Literature 5: Shimizu, T et al., J. Biomed. Mater. Res. 60, 110-117 (2002)
Non Patent Literature 6: Harimoto, M. et al., J. Biomed. Mater. Res. 62, 464-470 (2002)
Non Patent Literature 7: Shimizu, T., Yamato, M., Kikuchi, A. & Okano, T., Biomaterials 24, 2309-2316 (2003)

Non Patent Literature 8: Inflammation and Regeneration, Vol. 25, No. 3, 2005, pp. 158-159. the 26th Annual Meeting of the Japanese Society of Inflammation and Regeneration, "Toward Fusion of Study of Inflammation with Regenerative Medicine," Mitsuo OKANO

Summary of Invention

Object to be Solved by the Invention

In a polymer block comprised in the cell construct described in the Examples of Patent Literature 1, glutaraldehyde having strong toxicity to human bodies is used for crosslinking of polymers. A cell construct produced using such glutaraldehyde cannot be used for cell transplantation therapy because its toxicity to human bodies is a concern. A cell construct that does not contain a substance having toxicity to human bodies, such as glutaraldehyde, and can suppress the necrosis of the transplanted cells has not yet been discovered. As such, it has been desired to develop a cell construct that satisfies the aforementioned conditions and is suitable for use in cell transplantation therapy.

It is an object of the present invention to provide a cell construct for cell transplantation that does not contain a substance having cytotoxicity, such as glutaraldehyde, and suppresses the necrosis of the transplanted cells in the construct (namely, having a high cell survival rate). It is another object of the present invention to provide a biocompatible polymer block suitable for production of the aforementioned cell construct for cell transplantation. It is a further object of the present invention to provide a method for producing the aforementioned cell construct for cell transplantation and a method for producing the aforementioned biocompatible polymer block.

Means for Solving the Object

The scope of this invention is defined by the claims. Any references in the description to methods of treatment refer to the compounds, pharmaceutical compositions and medicaments of the present invention for use in a method for treatment of the human (or animal) body by therapy. As a result of intensive studies directed towards achieving the aforementioned objects, the present inventors have found that when a cell construct for cell transplantation is produced by using biocompatible polymer blocks that do not contain glutaraldehyde and at least one type of cells, and by disposing a plurality of biocompatible polymer blocks in gaps among a plurality of cells, a cell construct for cell transplantation that has achieved the aforementioned objects can be provided by using biocompatible polymer blocks wherein a tap density is 10 mg/$cm^3$ or more and 500 mg/$cm^3$ or less, or the value of the square root of the cross-sectional area / boundary length in the two-dimensional sectional image of the polymer block is 0.01 or more and 0.13 or less, wherein the size of one biocompatible polymer block is 20 $\mu$m or more and 200 $\mu$m or less, thereby completing the present invention.

The present invention provides a cell construct for cell transplantation comprising biocompatible polymer blocks that do not contain glutaraldehyde and at least one type of cells, wherein a plurality of biocompatible polymer blocks are disposed in gaps among a plurality of cells, and wherein the biocompatible polymer blocks have a tap density of 10 mg/$cm^3$ or more and 500 mg/$cm^3$ or less, or the value of the square root of the cross-sectional area / boundary length in the two-dimensional sectional image of the polymer block is 0.01 or more and 0.13 or less. The size of one biocompatible polymer block is 20 $\mu$m or more and 200 $\mu$m or less.

The present invention further provides a biocompatible polymer block that does not contain glutaraldehyde, wherein the biocompatible polymer block has a tap density of 10 mg/$cm^3$ or more and 500 mg/$cm^3$ or less, or the value of the square root of the cross-sectional area / boundary length in the two-dimensional sectional image of the polymer block is 0.01 or more and 0.13 or less. The size of the biocompatible polymer block is 20 $\mu$m or more and 200 $\mu$m or less.

Preferably, the size of one biocompatible polymer block is 50 $\mu$m or more and 120 $\mu$m or less.

Preferably, biocompatible polymers are crosslinked by heat, an ultraviolet ray or an enzyme.

Preferably, the biocompatible polymer block has a degree of cross-linkage of 6 or more, and also has a water absorption percentage of 300% or more.

Preferably, the biocompatible polymer block is obtained by crushing the porous body of a biocompatible polymer.

Preferably, the porous body of the biocompatible polymer is produced by a method comprising:

(a) a step of freezing a solution of the biocompatible polymer by a freezing treatment, in which the liquid temperature of the portion having the highest liquid temperature in the solution (highest internal liquid temperature) becomes "the melting point of a solvent - 3°C" or lower in an unfrozen state; and
(b) a step or freeze-drying the frozen biocompatible polymer obtained in the step (a).

Preferably, in the step (a), the solution of the biocompatible polymer is frozen by a freezing treatment, in which the liquid temperature of the portion having the highest liquid temperature in the solution (highest internal liquid temperature) becomes "the melting point of a solvent - 7°C" or lower in an unfrozen state.

Preferably, the porous body of the biocompatible polymer has the following properties (a) and (b):

(a) it has a porosity of 81% or more and 99.99% or less; and
(b) pores with a size of 20 to 200 μm have a space occupation percentage of 85% or more.

Preferably, the cell construct for cell transplantation has a thickness or a diameter of 400 μm or more and 3 cm or less.
Preferably, the cell construct for cell transplantation comprises biocompatible polymer blocks in an amount of 0.0000001 μg or more and 1 μg or less per cell.
Preferably, the biocompatible polymer is gelatin, collagen, elastin, fibronectin, pronectin, laminin, tenascin, fibrin, fibroin, entactin, thrombospondin, retronectin, polylactic acid, polyglycolic acid, a lactic acid-glycolic acid copolymer, hyaluronic acid, glycosaminoglycan, proteoglycan, chondroitin, cellulose, agarose, carboxymethyl cellulose, chitin, or chitosan.
Preferably, the biocompatible polymer is recombinant gelatin.
Preferably, the recombinant gelatin is represented by
the formula: A-[(Gly-X-Y) n]m-B
wherein A represents any given amino acid or amino acid sequence, B represents any given amino acid or amino acid sequence, an n number of X each independently represent an amino acid, an n number of Y each independently represent an amino acid, n represents an integer of 3 to 100, and m represents an integer of 2 to 10, wherein an n number of Gly-X-Y may be the same as or different from one another.
Preferably, the recombinant gelatin has (1) the amino acid sequence shown in SEQ ID NO: 1 or (2) an amino acid sequence showing homology of 80% or more with the amino acid sequence shown in SEQ ID NO: 1 and having biocompatibility.
Preferably, the cell construct for cell transplantation comprises an angiogenesis factor.
Preferably, the cells are selected from the group consisting of pluripotent cells, somatic stem cells, precursor cells, and mature cells.
Preferably, the cells are only non-vascular cells.
Preferably, the cells comprise both non-vascular cells and vascular cells.
Preferably, the cell construct for cell transplantation has a region in which the area of vascular cells in the central portion is larger than the area of vascular cells in the peripheral portion.
Preferably, the cell construct for cell transplantation has a region in which vascular cells in the central portion account for 60% to 100% of the total area of vascular cells.
Preferably, the cell construct for cell transplantation has a region in which the density of vascular cells in the central portion is $1.0 \times 10^{-4}$ cells/$\mu m^3$ or more.
Preferably, angiogenesis occurs inside the cell construct for cell transplantation.
Preferably, the biocompatible polymer block of the present invention is used for production of the cell construct for cell transplantation of the present invention.
The present invention further provides a reagent for producing the cell construct for cell transplantation of the present invention, which comprises the biocompatible polymer blocks of the present invention.
The present invention further provides a method for producing the cell construct for cell transplantation of the present invention, which comprises mixing the biocompatible polymer blocks of the present invention with at least one type of cells.
The present invention further provides a method for producing the porous body of a biocompatible polymer, which comprises:

(a) a step of freezing a solution of the biocompatible polymer by a freezing treatment, in which the liquid temperature of the portion having the highest liquid temperature in the solution (highest internal liquid temperature) becomes "the melting point of a solvent - 3°C" or lower in an unfrozen state; and
(b) a step or freeze-drying the frozen biocompatible polymer obtained in the step (a).

Preferably, in the step (a), the solution of the biocompatible polymer is frozen by a freezing treatment, in which the liquid temperature of the portion having the highest liquid temperature in the solution (highest internal liquid temperature) becomes "the melting point of a solvent - 7°C" or lower in an unfrozen state.

Advantageous Effects of Invention

The cell construct for cell transplantation of the present invention can be used for cell transplantation therapy because it does not contain a substance having cytotoxicity, such as glutaraldehyde, and it is also able to suppress the necrosis of the transplanted cells in the construct, and thus is excellent in terms of cell survival rate.

Brief Description of Drawings

[Figure 1] Figure 1 shows a difference among blocks in an *in vitro* ATP assay.
[Figure 2] Figure 2 shows a difference in the survival state of cells in hMSC mosaic cell masses in which the blocks of the present invention or comparative blocks are used (two weeks after the transplantation).
[Figure 3] Figure 3 shows the survival state of the transplanted cells in the block groups of the present invention. This figure shows the survival of the transplanted cells in hMSC mosaic cell masses and a difference caused by block size.
[Figure 4] Figure 4 shows angiogenesis in hMSC mosaic cell masses two weeks after the transplantation.
[Figure 5] Figure 5 shows angiogenesis in hMSC + hECFC mosaic cell masses two weeks after the transplantation.
[Figure 6] Figure 6 shows the space occupation percentages of individual pore sizes in porous bodies.
[Figure 7] Figure 7 shows the HE section images of CBE3 porous bodies, pore shapes, and highest internal liquid temperatures.
[Figure 8] Figure 8 shows a change over time in the highest internal liquid temperature at a shelf board temperature of -40°C (glass board: 2.2 mm).

Embodiments for Carrying out the Invention

Hereinafter, the embodiment of the present invention will be described in detail.

The cell construct for cell transplantation of the present invention is a cell construct for cell transplantation comprising biocompatible polymer blocks that do not contain glutaraldehyde and at least one type of cells, wherein a plurality of biocompatible polymer blocks are disposed in gaps among a plurality of cells, and wherein the biocompatible polymer blocks have a tap density of 10 mg/$cm^3$ or more and 500 mg/$cm^3$ or less, or a velue of the square root of the cross-sectional area / boundary length in the two-dimensional sectional image of the polymer block is 0.01 or more and 0.13 or less.

(1) Biocompatible polymer block

(1-1) Biocompatible polymer

The biocompatible polymer used in the present invention is not particularly limited by whether or not the polymer is degraded *in vivo* as long as it has affinity for organisms. It is preferred to be composed of a biodegradable material. A non-biodegradable material is specifically at least one material selected from the group consisting of polytetrafluoroethylene (PTFE), polyurethane, polypropylene, polyester, vinyl chloride, polycarbonate, acryl, stainless, titanium, silicone, and MPC (2-methaclylloyloxyethylphospholylcholine). A biodegradable material is specifically at least one material selected from the group consisting of polypeptide (for example, gelatin which is explained below), polylactic acid, polyglycolic acid, lactate/glycolate copolymer (PLGA), hyaluronic acid, glycosaminoglycan, proteoglycan, chondroitin, cellulose, agarose, carboxymethylcellulose, chitin, and chitosan. Among them, polypeptide is particularly preferable. In this context, these polymer materials may be given a contrivance to enhance cell adhesiveness. Methods such as [1] "coating of matrix surface with a cell-adhesive substrate (fibronectin, vitronectin, and laminin) or a cell adhesion sequence (RGD sequence, LDV sequence, REDV sequence, YIGSR sequence, PDSGR sequence, RYVVLPR sequence, LGTIPG sequence, RNIAEIIKDI sequence, IKVAV sequence, LRE sequence, DGEA sequence, and HAV sequence, indicated by single letter codes for amino acids) peptide", [2] "amination or cationization of matrix surface", and [3] "plasma treatment or corona discharge-based hydrophilic treatment of matrix surface" may be used as specific methods.

The type of the polypeptide is not particularly limited as long as it has biocompatibility. The polypeptide is preferably, for example, gelatin, collagen, elastin, fibronectin, ProNectin, laminin, tenascin, fibrin, fibroin, entactin, thrombospondin, or RetroNectin, most preferably gelatin, collagen, or atelocollagen. Natural gelatin or a recombinant gelatin is preferable as gelatin for use in the present invention. A recombinant gelatin is more preferable. In this context, the natural gelatin means gelatin formed from naturally derived collagen. The recombinant gelatin will be described later in the present specification.

The hydrophilicity value "1/IOB" value of the biocompatible polymer used in the present invention is preferably 0 to 1.0, more preferably 0 to 0.6, further preferably 0 to 0.4. IOB is an index for hydrophilicity and hydrophobicity based on the organic conception diagram representing the polarity/non-polarity of organic compounds proposed by Atsushi Fujita. The details thereof are described in, for example, "Pharmaceutical Bulletin", vol. 2, 2, pp. 163-173 (1954), "Journal of Japanese Chemistry" vol. 11, 10, pp. 719-725 (1957), and "Fragrance Journal", vol. 50, pp. 79-82 (1981). In short, this process involves assuming that methane (CH4) is the source of all organic compounds and all of the other compounds are methane derivatives, selecting a certain numerical value for each of the number of carbon atoms, substituents, modified moieties, rings and the like thereof, adding the scores to determine an organic value (OV) and an inorganic value (IV), and plotting this value on a diagram with the organic value on the X axis and the inorganic value on the Y axis. IOB on the organic conception diagram refers to the ratio of the inorganic value (IV) to the organic value (OV), i.e., "inorganic value (IV)/organic value (OV)", on the organic conception diagram. For the details of the organic conception diagram, see "Shinban Yuuki Gainenzu -Kiso to Ouyou- (New Edition, The Organic Conceptual Diagram, its Fundamentals and Applications in English)", (Yoshio Koda et al., Sankyo Publishing Co., Ltd., 2008)". In the present specification, hydrophilicity and hydrophobicity are indicated by "1/IOB" values, reciprocals of IOB. This notation represents that the smaller the "1/IOB" value becomes (the more the "1/IOB" value approaches 0), the more hydrophilic it is.

The "1/IOB" value of the polymer used in the present invention is set to within the range described above, whereby hydrophilicity is enhanced and water absorbability is enhanced. The resulting polymer is presumed to effectively act on retention of nutrients and, as a result, contribute to cell stabilization and viability in the three-dimensional cell construct (mosaic cell mass) of the present invention.

In the case where the biocompatible polymer used in the present invention is polypeptide, its index for hydrophilicity and hydrophobicity indicated by Grand average of hydropathicity (GRAVY) values is preferably from -9.0 to 0.3, more preferably from -7.0 to 0.0. The Grand average of hydropathicity (GRAVY) value can be obtained by the methods of "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31: 3784-3788 (2003)".

The GRAVY value of the polymer used in the present invention is set to within the range described above, whereby hydrophilicity is enhanced and water absorbability is enhanced. The resulting polymer is presumed to effectively act on retention of nutrients and, as a result, contribute to cell stabilization and viability in the three-dimensional cell construct (mosaic cell mass; cell mass having mosaic pattern) of the present invention.

(1-2) Cross-linking

The polymer biocompatible polymer used in the present invention may be cross-linked or may not be cross-linked. Those cross-linked are preferable. General known cross-linking methods include thermal cross-linking, cross-linking using an aldehyde (e.g., formaldehyde and glutaraldehyde), cross-linking using a condensing agent (carbodiimide, cyanamide, etc.), enzymatic cross-linking, photocrosslinking, UV cross-linking, hydrophobic interaction, hydrogen bond, or ionic interaction. In the present invention, cross-linking methods without use of glutaraldehyde are used. Preferably, in the present invention, cross-linking methods without use of aldehyde or condensing agent are used. The cross-linking method used in the present invention is preferably thermal cross-linking, UV cross-linking, or enzymatic cross-linking, and is particularly preferably thermal cross-linking.

In the case of performing cross-linking using an enzyme, the enzyme is not particularly limited as long as it has the effect of cross-linking between polymer materials. The cross-linking can be performed using preferably transglutaminase and laccase, most preferably transglutaminase. Specific examples of proteins that may be subjected to enzymatic cross-linking with transglutaminase are not particularly limited as long as they are proteins having a lysine residue and a glutamine residue. The transglutaminase may be derived from a mammal or may be derived from a microbe. Specific examples thereof include ACTIVA series manufactured by Ajinomoto Co., Inc., mammal-derived transglutaminase sold as reagents, for example, guinea pig liver-derived transglutaminase, goat-derived transglutaminase, and rabbit-derived transglutaminase manufactured by Oriental Yeast Co., ltd., Upstate USA Inc., or Biodesign International, and human-derived blood coagulation factor (Factor XIIIa, Haematologic Technologies, Inc.).

The reaction temperature for the cross-linking method (for example, thermal cross-linking) without using the cross-linking agent is not particularly limited as long as the cross-linking can be performed. The temperature is preferably -100°C to 500°C, more preferably 0°C to 300°C, further preferably 50°C to 300°C, further preferably 100°C to 250°C, further preferably 120°C to 200°C.

(1-3) Recombinant gelatin

The recombinant gelatin in the present invention means a polypeptide or a protein-like substance that is prepared by a gene recombination technique and has an amino acid sequence similar to gelatin. For the recombinant gelatin that can be used in the present invention, it is preferred to have repeats of the sequence represented by Gly-X-Y (X and Y each independently represent any amino acid) characteristic of collagen (a plurality of Gly-X-Y sequences may be the same as or different from each other). Preferably, two or more sequences of cell adhesion signals are contained in a molecule. A recombinant gelatin having an amino acid sequence derived from a partial amino acid sequence of collagen can be used as the recombinant gelatin used in the present invention. For example, those described in EP1014176, US6992172, WO2004/85473, and WO2008/103041 can be used, though the recombinant gelatin is not limited to them. A preferable recombinant gelatin used in the present invention is a recombinant gelatin having the following aspect:

The recombinant gelatin used in the present invention is excellent in biocompatibility based on the original performance of natural gelatin, is free from concerns about Bovine Spongiform Encephalopathy (BSE) or the like because of being not naturally derived, and is also excellent in non-infectious properties. Moreover, since the recombinant gelatin used in the present invention is homogeneous compared with natural one and its sequence is determined, it can be designed precisely with a little variation in strength or degradability depending on cross-linking or the like described later.

The molecular weight of the recombinant gelatin is preferably from 2 KDa to 100 KDa, more preferably from 2.5 KDa to 95 KDa, further preferably from 5 KDa to 90 KDa, most preferably from 10 KDa to 90 KDa.

The recombinant gelatin has repeats of the sequence represented by Gly-X-Y characteristic of collagen. In this context, a plurality of Gly-X-Y sequences may be the same as or different from each other. In Gly-X-Y, Gly represents glycine, and X and Y each represent any amino acid (preferably, any amino acid other than glycine). The GXY sequence characteristic of collagen is a very specific partial structure in the amino acid composition and sequence of gelatin/collagen, compared with other proteins. In this moiety, glycine accounts for approximately 1/3 of the whole and appears at a rate of one out of three amino acids in the amino acid sequence. Glycine is the simplest amino acid. Its position in the molecular chain is less restricted, and glycine makes a significant contribution to the regeneration of the helix structure during gelatinization. It is preferred that imino acids (proline or oxyproline) should be included in large amounts in the amino acids represented by X and Y and account for 10% to 45% of all the amino acids. It is preferred that preferably 80% or more, more preferably 95% or more, most preferably 99% or more of the amino acids in the sequence should be the GXY repeat structures.

In general gelatin, as to the polar amino acids, those having an electric charge and those uncharged are present at a 1:1 ratio. In this context, the polar amino acids specifically refer to cysteine, aspartic acid, glutamic acid, histidine, lysine, asparagine, glutamine, serine, threonine, tyrosine, and arginine. Of them, polar uncharged amino acids refer to cysteine, asparagine, glutamine, serine, threonine, and tyrosine. The ratio of the polar amino acids is 10 to 40%, preferably 20 to 30%, to all amino acids constituting the recombinant gelatin used in the present invention. In addition, it is preferred that the ratio of uncharged amino acids to the polar amino acids should be from 5% to less than 20%, preferably less than 10%. It is further preferred that any one amino acid, preferably two or more amino acids which are selected from serine, threonine, asparagine, tyrosine, and cysteine, should not be contained in the sequence.

In general, minimal amino acid sequences that function as cell adhesion signals in polypeptides are known (e.g., "Medicina Philosophica", Vol. 9, No. 7 (1990), p. 527, Nagai Shoten Co., Ltd.). It is preferred that the recombinant gelatin used in the present invention should have two or more of these cell adhesion signals in a molecule. Specific sequences are preferably RGD sequences, LDV sequences, REDV sequences, YIGSR sequences, PDSGR sequences, RYVVLPR sequences, LGTIPG sequences, RNIAEIIKDI sequences, IKVAV sequences, LRE sequences, DGEA sequences, and HAV sequences, more preferably RGD sequences, YIGSR sequences, PDSGR sequences, LGTIPG sequences, IKVAV sequences, and HAV sequences, particularly preferably RGD sequences, indicated by single letter codes for amino acids, in terms that many types of cell can adhere thereto. Of the RGD sequences, an ERGD sequence is preferable. The amount of substrates produced by the cells can be improved by using the recombinant gelatin having cell adhesion signals. In the case of, for example, chondrogenic differentiation using mesenchymal stem cells as the cells, the production of glycosaminoglycan (GAG) can be improved.

For the arrangement of the RGD sequences in the recombinant gelatin used in the present invention, it is preferred that the number of amino acids between the RGD sequences should be between 0 and 100, preferably between 25 and 60, and should not be uniformly determined.

From the viewpoint of cell adhesion/growth, the content of this minimal amino acid sequence is preferably 3 to 50 sequences, more preferably 4 to 30 sequences, particularly preferably 5 to 20 sequences, most preferably 12 sequences, per protein molecule.

In the recombinant gelatin used in the present invention, the ratio of the RGD motifs to the total number of the amino acids is preferably at least 0.4%. In the case where the recombinant gelatin contains 350 or more amino acids, it is preferred that each stretch of 350 amino acids should contain at least one RGD motif. The ratio of the RGD motifs to the total number of the amino acids is more preferably at least 0.6%, further preferably at least 0.8%, further preferably at least 1.0%, further preferably at least 1.2%, most preferably at least 1.5%. The number of the RGD motifs within the recombinant gelatin is preferably at least 4, more preferably 6, further preferably 8, further preferably from 12 to 16, per 250 amino acids. The ratio of the RGD motifs of 0.4% corresponds to at least one RGD sequence per 250 ammo acids. Since the number of the RGD motifs is an integer, gelatin consisting of 251 amino acids must contain at least two RGD sequences in order to satisfy the feature of 0.4%. Preferably, the recombinant gelatin of the present invention contains at least two RGD sequences per 250 amino acids, more preferably at least three RGD sequences per 250 amino acids, further preferably at least four RGD sequences per 250 amino acids. In a further aspect, the recombinant gelatin of the present invention comprises at least 4 RGD motifs, preferably 6, more preferably 8, further preferably 12 to 16 RGD motifs.

Moreover, the recombinant gelatin may be partially hydrolyzed.

It is preferred that the recombinant gelatin used in the present invention is represented by a formula : A[(Gly-X-Y)n]mB. an n number of X each independently represent an amino acid, an n number of Y each independently represent an amino acid. m is preferably 2 to 10, more preferably 3 to 5. n is preferably 3 to 100, more preferably 15 to 70, most preferably 50 to 65. A represents any given amino acid or amino acid sequence, B represents any given amino acid or amino acid sequence, an n number of X each independently represent an amino acid, an n number of Y each independently represent an amino acid.

It is more preferred that the recombinant gelatin used in the present invention is represented by a formula: Gly-Ala-Pro-[(Gly-X-Y)63]3-Gly wherein 63 number of X independently represent an amino acid, an 63 number of Y each independently represent an amino acid. 63 number of Gly-X-Y may be the same as or different from one another.

It is preferred that a plurality of naturally occurring collagen sequence units should be bonded to repeat units. In this context, the naturally occurring collagen may be any naturally occurring collagen and is preferably type-I, type-II, type-III, type-IV, and type-V collagens, more preferably type-I, type-II, and type-III collagens. In another embodiment, the origin of the collagen is preferably a human, cattle, a pig, a mouse, or a rat, more preferably a human.

The isoelectric point of the recombinant gelatin used in the present invention is preferably 5 to 10, more preferably 6 to 10, further preferably 7 to 9.5.

Preferably, the recombinant gelatin is not deaminated.

Preferably, the recombinant gelatin does not have telopeptide.

Preferably, the recombinant gelatin is a substantially pure collagen material which was prepared from a nucleic acid encoding natural collagen.

The recombinant gelatin used in the present invention is particularly preferably a recombinant gelatin having any of the followings:

(1) the amino acid sequence represented by SEQ ID NO: 1; or
(2) an amino acid sequence having 80% or higher (more preferably 90% or higher, most preferably 95% or higher) homology to the amino acid sequence represented by SEQ ID NO: 1 and having biocompatibility.

The recombinant gelatin used in the present invention can be produced by a gene recombination technique known by those skilled in the art and can be produced according to a method described in, for example, EP1014176A2, US6992172, WO2004-85473, or WO2008/103041. Specifically, a gene encoding the amino acid sequence of the predetermined recombinant gelatin is obtained, and this is incorporated in an expression vector to prepare a recombinant expression vector, which is then introduced in appropriate hosts to prepare transformants. The obtained transformants are cultured in an appropriate medium, whereby the recombinant gelatin is produced. Thus, the produced recombinant gelatin can be collected from the cultures to prepare the recombinant gelatin used in the present invention.

(1-4) Biocompatible polymer block

In the present invention, a block (mass) containing the above described biocompatible polymer is used.

The shape of the biocompatible polymer block of the present invention is not particularly limited. The shape of the present biocompatible polymer block satisfies any one or more conditions that a tap density is 10 $mg/cm^3$ or more and 500 $mg/cm^3$ or less, and that the value of the square root of the cross-sectional area / boundary length in the two-dimensional sectional image of the biocompatible polymer block is 0.01 or more and 0.13 or less.

The tap density of the biocompatible polymer block of the present invention is 10 $mg/cm^3$ or more and 500 $mg/cm^3$ or less, preferably 20 $mg/cm^3$ or more and 400 $mg/cm^3$ or less, more preferably 40 $mg/cm^3$ or more and 220 $mg/cm^3$ or less, and even more preferably 50 $mg/cm^3$ or more and 150 $mg/cm^3$C or less.

The tap density is a value indicating how many blocks can be filled in a certain volume. As the value of the tap density decreases, it indicates that the blocks cannot be densely filled, namely, the structure of the blocks is found to be complicated. The tap density of biocompatible polymer blocks is considered to indicate the complexity of the surface structure of the biocompatible polymer blocks and the amount of voids formed when such biocompatible polymer blocks are gathered to form an aggregate. As the tap density decreases, voids among the polymer blocks increase, and thus, regions into which cells are engrafted also increase. In addition, because of a not-too-small tap density, biocompatible polymer blocks can be adequately present among cells, and when a cell construct for cell transplantation is formed with such biocompatible polymer blocks, it becomes possible to deliver nutrient into the construct. Accordingly, it is considered that the tap density is preferably in the aforementioned range.

The tap density used in the present description can be measured as follows. For the measurement of the tap density, a vessel (a cylindrical vessel with a diameter of 6 mm and a length of 21.8 mm; volume: 0.616 $cm^3$) (hereinafter referred to as a "cap") is prepared. First, the mass of a cap alone is measured. Thereafter, a funnel is fixed to the cap, blocks are then supplied into the cap through the funnel, so that the blocks can be accumulated in the cap. After a sufficient amount of blocks have been placed in the cap, the cap portion is slammed against a hard stuff such as a desk 200 times. Thereafter, the funnel is removed from the cap, and the blocks are then leveled using a spatula. The mass of one level cap of blocks is measured. The mass of the blocks alone is calculated based on the difference between the mass of the cap alone and the mass of the one level cap of blocks. The mass of the blocks alone is divided by the volume of the cap to obtain a tap density.

The size of one biocompatible polymer block of the present invention is 20 μm or more and 200 μm or less. It is more preferably 20 μm or more and 120 μm or less, and even more preferably 50 μm or more and 120 μm or less.

Preferably, the tap density of the biocompatible polymer blocks is 10 $mg/cm^3$ or more and 400 $mg/cm^3$ or less, and the size of one polymer block is 20 μm or more and 120 μm or less.

The "square root of cross-sectional area / boundary length" of the biocompatible polymer block of the present invention is 0.01 or more and 0.13 or less, preferably 0.02 or more and 0.12 or less, more preferably 0.03 or more and 0.115 or less, and even more preferably 0.05 or more and 0.09 or less.

As with tap density, the "square root of cross-sectional area / boundary length" of the biocompatible polymer block is considered to indicate the complexity of the surface structure of the biocompatible polymer blocks and the amount of voids formed when such biocompatible polymer blocks are gathered to form an aggregate. As the "square root of cross-sectional area / boundary length" decreases, voids among the biocompatible polymer blocks increase, and thus, regions into which cells are engrafted also increase. In addition, because of a not-too-small square root of cross-sectional area / boundary length, biocompatible polymer blocks can be adequately present among cells, and when a cell construct for cell transplantation is formed with such biocompatible polymer blocks, it becomes possible to deliver nutrient into the construct. Accordingly, it is considered that the square root of cross-sectional area / boundary length is preferably in the aforementioned range.

The "square root of cross-sectional area / boundary length" of the biocompatible polymer block in a two-dimensional sectional image can be obtained by producing a cross-sectional specimen of the biocompatible polymer block and then examining the cross-sectional structure thereof. For instance, first, a cross-sectional structure of the biocompatible polymer block is used as a thin sliced specimen (e.g., a HE-stained specimen). At this time, the biocompatible polymer block may be used alone, or the cross-sectional structure of a cell construct comprising the biocompatible polymer blocks and cells may also be observed. The cross-sectional area and boundary length of one biocompatible polymer block are obtained, and thereafter, the "square root of cross-sectional area / boundary length" is calculated. Such values are measured over 10 or more sites, and the "square root of cross-sectional area / boundary length" can be obtained as a mean value of the obtained values.

The size of one biocompatible polymer block of the present invention is 20 μm or more and 200 μm or less, and more preferably 50 μm or more and 120 μm or less. By setting the size of one biocompatible polymer block in the aforementioned range, a more excellent cell survival rate can be achieved. It is to be noted that the size of one biocompatible polymer block does not mean that a mean value of the sizes of a plurality of biocompatible polymer blocks is in the aforementioned range, but it means the size of individual single biocompatible polymer block obtained by sieving a plurality of biocompatible polymer blocks.

The degree of cross-linkage of the biocompatible polymer block of the present invention is not particularly limited. It is preferably 6 or more, more preferably 6 or more and 30 or less, even more preferably 6 or more and 25 or less, and particularly preferably 8 or more and 22 or less.

The method for measuring the degree of cross-linkage (the number of cross-linkages per molecule) of the polymer block is not particularly limited, and the degree of cross-linkage can be measured, for example, by a TNBS (2,4,6-trinitrobenzenesulfonic acid) method described in the after-mentioned Examples. Specifically, a polymer block, a $NaHCO_3$ aqueous solution, and a TNBS aqueous solution are mixed, and the mixed solution is then reacted at 37°C for 3 hours. Thereafter, the reaction is terminated to obtain a sample. At the same time, the reaction is terminated immediately after the mixing of a polymer block, a $NaHCO_3$ aqueous solution, and a TNBS aqueous solution, so as to obtain a blank. Thereafter, the absorbance (345 nm) of each of the sample and the blank, which have been diluted with pure water, is measured, and the degree of cross-linkage (the number of cross-linkages per molecule) can be calculated according to the following (Formula 1) and (Formula 2).

$$\text{(Formula 1)}\ (As\text{-}Ab)/14600 \times V/w$$

(Formula 1) shows the amount (equimolar amount) of lysine per g of polymer block.

(In the above formula, As represents the absorbance of the sample, Ab represents the absorbance of the blank, V represents the amount of the reaction solution (g), and w represents the mass (mg) of the polymer block.)

$$\text{(Formula 2) 1- (sample (Formula 1) / uncrosslinked polymer (Formula 1))} \times 34$$

(Formula 2) shows the number of cross-linkages per molecule.

The water absorption percentage of the biocompatible polymer block of the present invention is not particularly limited. It is preferably 300% or more, more preferably 400% or more, even more preferably 500% or more, particularly preferably 700% or more, and most preferably 800% or more. The upper limit of the water absorption percentage is not particularly limited. In general, it is 4000% or less, or 2000% or less.

The method for measuring the water absorption percentage of the biocompatible polymer block is not particularly limited. The water absorption percentage of the biocompatible polymer block can be measured, for example, by the method described in the after-mentioned Examples. Specifically, approximately 15 mg of the biocompatible polymer blocks are filled in a bag made of nylon mesh with a size of 3 cm × 3 cm at 25°C, and the blocks are then swollen in ion exchange water for 2 hours. Thereafter, the resulting blocks are air-dried for 10 minutes. The mass thereof is measured at individual stages, and then, the water absorption percentage can be obtained according to the following (Formula 3).

(Formula 3)

$$\text{Water absorption percentage} = (w2 - w1 - w0) / w0$$

(In the formula, w0 represents the mass of the material before water absorption, w1 represents the mass of the empty bag after water absorption, and w2 represents the mass of the entire bag that contains the material after water absorption.)

(1-5) Method for producing biocompatible polymer block

The method for producing the biocompatible polymer block is not particularly limited, as long as the produced biocompatible polymer block satisfies the conditions described in (1-4) above. For example, the porous body of a biocompatible polymer is crushed using a crushing machine (New Power Mill, etc.), so as to obtain a biocompatible polymer block that satisfies the conditions described in (1-4) above.

As a "porous body" used in the present invention, for example, a material that has a plurality of pores with a size of "10 μm or more and 500 μm or less" therein when the material is a 1 mm square material, wherein the pores account for 50% or more of the total volume of the material, can be preferably used. In such a material, the aforementioned internal pores may be communicated with one another, and a part of or the entire pores may have an opening on the surface of the material.

Upon production of the porous body of a biocompatible polymer, by allowing the production method to comprise a freezing step in which the liquid temperature of the portion having the highest liquid temperature in the solution (highest internal liquid temperature) becomes "the melting point of a solvent - 3°C" or lower in an unfrozen state, the formed ice has a spherical shape. By drying the ice after the aforementioned freezing step, a porous body having spherical isotropic pores (spherical pores) can be obtained. On the other hand, if the solution is frozen without the freezing step in which the liquid temperature of the portion having the highest liquid temperature in the solution (highest internal liquid temperature) becomes "the melting point of a solvent - 3°C" or lower in an unfrozen state, the formed ice has a columnar/planar shape. When the ice is dried after this step, a porous body having uniaxially or biaxially long, columnar or planar pores (columnar/planar pores) can be obtained.

In the present invention, with regard to the shape of pores in a porous body, spherical pores are preferable, rather than columnar/planar pores. In addition, it is more preferable that spherical pores account for 50% or more of the entire pores.

In the present invention, the porous body of a biocompatible polymer can be preferably produced by a method comprising:

(a) a step of freezing a solution of the biocompatible polymer by a freezing treatment, in which the liquid temperature of the portion having the highest liquid temperature in the solution (highest internal liquid temperature) becomes "the melting point of a solvent - 3°C" or lower in an unfrozen state; and

(b) a step or freeze-drying the frozen biocompatible polymer obtained in the step (a). This is because the percentage of spherical pores to the entire pores can be set at 50% or more by the above described steps.

More preferably, in the above described step (a), the solution of the biocompatible polymer can be frozen by a freezing treatment, in which the liquid temperature of the portion having the highest liquid temperature in the solution (highest internal liquid temperature) becomes "the melting point of a solvent - 7°C" or lower in an unfrozen state. This is because the percentage of spherical pores to the entire pores can be set at 80% or more by this step.

The mean pore size of pores in a porous body can be obtained by observing the cross-sectional structure of the porous body. First, the cross-sectional structure of the porous body is prepared in the form of a thin sliced specimen (for example, a HE-stained specimen). Subsequently, among walls formed with polymers, clear projections are connected with projections nearest thereto, so as to make the shapes of pores clear. The area of the thus obtained each pore is measured, and the diameter of a circle, which is obtained when the measured area is calculated relative to circle, is calculated. The obtained circle diameter is defined as a pore size, and a mean value of 20 or more pore sizes can be defined as a mean pore size.

In the present description, the space occupation percentage of a certain pore size means the percentage of the volume of pores having the certain pore size to the volume of the porous body. Specifically, the space occupation percentage can be obtained by dividing the area of pores having a certain pore size by the total area, based on a two-dimensional image. Moreover, as a section image used herein, a section image with a real scale of 1.5 mm can be used.

The space occupation percentage of pores with a pore size of 20 μm to 200 μm in a porous body is preferably 83% or more and 100% or less, more preferably 85% or more and 100% or less, even more preferably 90% or more and 100% or less, and particularly preferably 95% or more and 100% or less.

The space occupation percentage of pores with a pore size of 30 μm to 150 μm in a porous body is preferably 60% or more and 100% or less, more preferably 70% or more and 100% or less, even more preferably 80% or more and 100% or less, and particularly preferably 90% or more and 100% or less.

The phases "the space occupation percentage of pores with a pore size of 20 μm to 200 μm in a porous body" and "the space occupation percentage of pores with a pore size of 30 μm to 150 μm in a porous body" mean that a pore size distribution in the porous body can be set in a predetermined range. That is, when polymer blocks with a size of 20 μm to 200 μm are obtained by crushing the concerned porous body, this pore size is a size close to one polymer block size, and consequently, in a porous body in which the percentage of this pore size is high, the structure of polymer blocks obtained after the porous body has been crushed becomes complicated, and this results in a reduction in the tap density or the "square root of cross-sectional area / boundary length."

With regard to the shape of a pore, the long axis and the short axis are measured with regard to individual pores, and the "long axis / short axis" is calculated based on each of the obtained values. When such "long axis / short axis" is 1 or more and 2 or less, the pore is considered to be a spherical pore, and when it is 3 or more, the pore is considered to be a columnar/planar pore.

Using apparent density (p) and true density (pc), the porosity of the porous body of the present invention can be obtained by the formula: porosity (P) = 1-p/pc (%). The apparent density (p) can be calculated from dry mass and volume, whereas the true density (pc) can be obtained by a specific gravity bottle method using a Hubbard bottle. The porosity of the polymer porous body of the present invention is preferably 81% or more and 99.99% or less, and more preferably 95.01% or more and 99.9% or less.

(1-6) Intended use of biocompatible polymer block

As described later in the present description, the biocompatible polymer blocks of the present invention are mixed with at least one type of cells, so that the cell construct for cell transplantation of the present invention can be produced. That is to say, the biocompatible polymer block of the present invention is useful as a reagent for producing the cell construct for cell transplantation of the present invention.

(2) Cells

Any given cells can be used in the present invention, as long as they are used in cell transplantation, which is the purpose of the cell construct of the present invention. The type thereof is not particularly limited. Moreover, the cells used may be of one type, or a combination of a plurality of types of cells may also be used. Furthermore, the cells used are preferably animal cells, more preferably vertebrate-derived cells, and particularly preferably human-derived cells. The type of the vertebrate-derived cells (particularly, human-derived cells) may be any of pluripotent cells, somatic stem cells, precursor cells, and mature cells. For example, ES cells, GS cells, or iPS cells can be used as pluripotent cells. For example, mesenchymal stem cells (MSCs), hematopoietic stem cells, amnion cells, cord blood cells, bone marrow-derived cells, cardiac muscle stem cells, fat-derived stem cells, or neural stem cells can be used as somatic stem cells. For example, cells derived from the skin, dermis, epidermis, muscle, cardiac muscle, nerve, bone, cartilage, endothelium, brain, epithelium, heart, kidney, liver, pancreas, spleen, oral cavity, cornea, bone marrow, cord blood, amnion, or hair can be used as precursor cells and mature cells. For example, ES cells, iPS cells, MSCs, cartilage cells, osteoblasts, osteoprogenitor cells, mesenchyme cells, myoblasts, cardiac muscle cells, cardiac myoblasts, nerve cells, hepatic cells, beta cells, fibroblasts, corneal endothelial cells, vascular endothelial cells, corneal epithelial cells, amnion cells, cord blood cells, bone marrow-derived cells, or hematopoietic stem cells can be used as human-derived cells. Moreover, the origin of the cells may be either autologous cells or heterologous cells.

Examples of cells that can be preferably used for heat diseases such as severe heart failure and severe myocardial infarction include autologously or heterologously extracted cardiac muscle cells, smooth muscle cells, fibroblasts, skeletal muscle-derived cells (particularly, satellite cells), and bone marrow cells (particularly, bone marrow cells differentiated into cardiac muscle-like cells). Furthermore, cells to be transplanted can be selected appropriately for other organs. Examples of such cell transplantation include transplantation of neural precursor cells or cells capable of being differentiated into nerve cells into a cerebral ischemia/cerebral infarction site, and transplantation of vascular endothelial cells or cells capable of being differentiated into vascular endothelial cells into a myocardial infarction/skeletal muscle ischemia site.

Further examples of the cells include cells for use in cell transplantation for diabetic organ damage. Specific examples thereof include cells for a cell transplantation treatment method variously studied on diseases such as kidney diseases, pancreas diseases, peripheral nerve diseases, eye diseases, or hematogenous disorder in the extremities. Specifically, an attempt to transplant insulin-secreting cells to the pancreas having the reduced ability to secrete insulin, transplantation of bone marrow-derived cells for hematogenous disorder in the extremities, or the like has been studied, and such cells can be used.

Moreover, as described later in the present description, vascular cells can also be used in the present invention. In the present description, the vascular cells mean cells related to angiogenesis and cells constituting blood vessels or blood, and precursor cells or somatic stem cells capable of being differentiated into these cells. In this context, the vascular cells do not include pluripotent cells such as ES cells, GS cells, or iPS cells, or cells that are not spontaneously differentiated into cells constituting blood vessels or blood, such as mesenchymal stem cells (MSCs). The vascular cells are preferably cells constituting blood vessels. Examples of the cells constituting blood vessels among vertebrate-derived cells (particularly, human-derived cells) can preferably include vascular endothelial cells and vascular smooth muscle cells. The vascular endothelial cells include both venous endothelial cells and arterial endothelial cells. Vascular endothelial precursor cells can be used as precursor cells for the vascular endothelial cells. Vascular endothelial cells and vascular endothelial precursor cells are preferable. Blood cells can be used as cells constituting blood, and white blood cells such as lymphocytes or neutrophils, monocytes, or hematopoietic stem cells as their stem cells can be used.

In the present description, non-vascular cells mean cells other than the vascular cells described above. For example, ES cells, iPS cells, mesenchymal stem cells (MSCs), cardiac muscle stem cells, cardiac muscle cells, fibroblasts, myoblasts, cartilage cells, myoblasts, hepatic cells, or nerve cells can be used. Preferably, mesenchymal stem cells (MSCs), cartilage cells, myoblasts, cardiac muscle stem cells, cardiac muscle cells, hepatic cells, or iPS cells can be used. Mesenchymal stem cells (MSCs), cardiac muscle stem cells, cardiac muscle cells, or myoblasts are more preferable.

(3) Cell constructs

In the present invention, the above described biocompatible polymer blocks and the above described cells are used, and the polymer blocks are three-dimensionally disposed in gaps among the cells in a mosaic pattern, whereby the cell construct can have a thickness suitable for cell transplantation. In addition, the biocompatible polymer blocks and the cells are three-dimensionally disposed in a mosaic pattern, whereby a three-dimensional cell construct in which the cells are uniformly present can be formed to enable nutrition delivery into the three-dimensional cell construct from outside. As a result, when cell transplantation is performed using the cell construct for cell transplantation of the present invention, transplantation with the necrosis of the transplanted cells is suppressed, and thus, transplantation can be achieved. In this context, the "suppression of necrosis" means that the degree of necrosis is low compared with the case where only the cells are transplanted without being contained in the cell construct of the present invention.

In the cell construct for cell transplantation of the present invention, a plurality of polymer blocks are disposed in gaps among a plurality of the cells. In this context, the "gaps among the cells" do not have to be closed spaces created by the constituent cells, and need only to be flanked by the cells. It is not required that all the cells should create such gaps thereamong. There may be a region in which the cells are in contact with one another. The distance of each gap between the cells via the polymer block(s), i.e., the distance of the gap from a certain cell to a selected cell located nearest from the certain cell, is not particularly limited and is preferably, the size of polymer block(s). The preferable distance is also in the range of preferable sizes of the polymer block(s).

Moreover, the polymer blocks according to the present invention are flanked by the cells in the constitution. It is not required that all the polymer blocks should be flanked by the cells. There may be a region in which the polymer blocks are in contact with each other. The distance between the polymer blocks via the cell(s), i.e., the distance from a certain polymer block to a selected polymer block located nearest from the certain polymer block, is not particularly limited and is preferably the size of one cell used or a cell mass containing a cell population, for example, from 10 μm

to 1000 μm, preferably from 10 μm to 100 μm, more preferably from 10 μm to 50 μm.

In the present description, the phrase "uniformly present" is used, as described in a "three-dimensional cell construct in which the cells are uniformly present". However, this phrase does not mean complete uniformity but means that the cells are distributed in a range that achieves the effects of the present invention, i.e., enables nutrition delivery into the three-dimensional cell construct from outside, and prevents the necrosis of the transplanted cells.

With regard to the thickness or diameter of the cell construct for cell transplantation of the present invention, the cell construct may have a desired thickness. The lower limit is preferably 215 μm or more, more preferably 400 μm or more, and most preferably 730 μm or more. The upper limit of such a thickness or a diameter is not particularly limited. As a general range used, it is preferably 3 cm or less, more preferably 2 cm or less, and even more preferably 1 cm or less. Moreover, the thickness or diameter of the cell construct is preferably 400 μm or more and 3 cm or less, more preferably 500 μm or more and 2 cm or less, and even more preferably 720 μm or more and 1 cm or less.

In the cell construct for cell transplantation of the present invention, regions consisting of the polymer blocks and regions consisting of the cells are preferably disposed in a mosaic pattern. In the present description, the "thickness or diameter of the cell construct" represents the following: when a certain point A in the cell construct is selected, the length of a line segment that is located on a straight line passing through the point A and partitions the cell construct to give the shortest distance between the cell construct and the outside world is defined as a line segment A. The point A is selected such that the line segment A becomes longest in the cell construct. The length of this longest line segment A is defined as the "thickness or diameter of the cell construct".

Moreover, in the case of using the cell construct of the present invention as a cell construct before fusion or as a cell construct before addition of second polymer blocks in the after-mentioned method for producing the cell construct for cell transplantation of the present invention, the range of the thickness or diameter of the cell construct is preferably 10 μm or more and 1 cm or less, more preferably 10 μm or more and 2000 μm or less, even more preferably 15 μm or more and 1500 μm or less, and most preferably 20 μm or more and 1300 μm or less.

In the cell construct for cell transplantation of the present invention, the ratio between the cells and the polymer blocks is not particularly limited, and it is a ratio of the polymer blocks per cell that is preferably 0.0000001 μg or more and 1 μg or less, more preferably 0.000001 μg or more and 0.1 μg or less, even more preferably 0.00001 μg or more and 0.01 μg or less, and most preferably 0.00002 μg or more and 0.006 μg or less. The cells can be more uniformly present by adopting the above described range. Moreover, the cells can exhibit effects during use in the application described above by adopting the above described range as the lower limit. Components arbitrarily present in the polymer blocks can be supplied to the cells by adopting the above described range as the upper limit. In this context, examples of the components in the polymer blocks include, but not particularly limited to, components contained in a medium described later.

The cell construct for cell transplantation of the present invention may further comprise an angiogenesis factor. In this context, examples of the angiogenesis factor can preferably include a basic fibroblast growth factor (bFGF), a vascular endothelial growth factor (VEGF), and a hepatocyte growth factor (HGF). A method for producing the cell construct for cell transplantation comprising such an angiogenesis factor is not particularly limited. For example, the cell construct can be produced by using polymer blocks impregnated with an angiogenesis factor. From the viewpoint of promotion of angiogenesis, the cell construct for cell transplantation of the present invention preferably comprises an angiogenesis factor.

An example of the cell aggregate for cell transplantation of the present invention is a cell aggregate for cell transplantation comprising non-vascular cells and vascular cells, wherein the cell aggregate for cell transplantation satisfies at least one of the following requirements:

(a) the area of the vascular cells in the central portion of the cell aggregate is larger than the area of the vascular cells in the peripheral portion, and
(b) the cell aggregate has a region in which the density of the vascular cells in the central portion is $1.0 \times 10^{-4}$ cells/$\mu m^3$ or more.

The cell aggregate for cell transplantation of the present invention preferably satisfies both of the requirements (a) and (b), and it is also preferable that the cell aggregate for cell transplantation of the present invention has a region in which vascular cells in the central portion account for 60% to 100% of the total area of vascular cells.

The cell construct for cell transplantation of the present invention comprising non-vascular cells can be preferably used. Moreover, the cell construct for cell transplantation of the present invention containing only non-vascular cell as its constituent cells can also be preferably used. The cell construct for cell transplantation of the present invention containing only non-vascular cells as the cells is capable of forming blood vessels at its transplantation site after transplantation. Furthermore, in a case where the cell construct for cell transplantation of the present invention contains two or more types of constituent cells comprising both non-vascular cells and vascular cells, this cell construct is more capable of forming blood vessels, and thus, it is more preferable than the cell construct containing only non-vascular cells as the constituent cells.

Furthermore, in a case where the cell construct for cell transplantation of the present invention contains two or more types of constituent cells, wherein the area of the vascular cells in the central portion of the cell construct is larger than the area of the vascular cells in the peripheral portion, this cell construct is much more capable of forming blood vessels, and thus, it is much more preferable. In this context, the phrase "the area of the vascular cells in the central portion of the cell construct is larger than the area of the vascular cells in the peripheral portion" specifically means that when any given 2-μm thin sliced specimens are produced, a specimen having the aforementioned region is present. In this context, the central portion of the cell construct refers to an area corresponding to a distance up to 80% from the center in the distance from the center to the surface of the cell construct. The peripheral portion of the cell construct refers to an area from the position of 80% from the center to the surface of the construct. In this context, the central portion of the cell construct is defined as follows.

With regard to any given cross section that passes through the center of the cell construct, a radius X is determined such that, when the center of a circle with the radius X is moved around the cross section along with the external margin of the cross section, the area of a portion except for the overlap between the moved circle and the cross section accounts for 64% of the cross-sectional area of the cross section. The center of a circle with the determined radius X is moved therearound, and a portion except for the overlap between the moved circle and the cross section is defined as the central portion of the cell construct. At this time, a cross section that gives the largest cross-sectional area is most preferable. With regard to the center of the cell construct, a radius Y is determined such that, when the center of a circle with the radius Y is moved around the cross section along with the external margin of a cross section that gives the largest cross-sectional area, a portion except for the overlap between the moved circle and the cross section can be set as a point. The center of a circle with the determined radius Y is moved therearound, and a portion except for the overlap between the moved circle and the cross section is defined as the center of the cell construct. When the center of the cell construct is not determined as a point, but becomes a line, or when a plurality of lines are present, a point that divides each line into two equal parts is defined as the center of the cell construct.

Specifically, the cell construct of the present invention preferably has a region in which the percentage of the vascular cells in the central portion is preferably 60% to 100%, more preferably 65% to 100%, even more preferably 80% to 100%, and further preferably 90% to 100%, to the total area of the vascular cells. In this context, the phrase "the percentage of the vascular cells in the central portion is 60% to 100% to the total area of the vascular cells" specifically means that when any given 2-μm thin sliced specimens are produced, a specimen with a region having the aforementioned percentage is present. Angiogenesis can be further promoted by adopting the aforementioned range.

The percentage of the vascular cells in the central portion can also be calculated, for example, by: staining vascular cells to be measured when a thin sliced specimen is prepared; determining an average value of the color density (strength) of the central portion using the image processing software ImageJ; calculating area × strength for the central portion; further determining an average value of color density (strength) as a whole; calculating area × strength as a whole; and determining the ratio of the value of area × strength for the central portion to the value of area × strength as a whole. In this context, a known staining method can be used appropriately as a method for staining the vascular cells. For example, in the case of using human endothelial progenitor cells (hECFC) as the cells, an anti-CD31 antibody can be used.

It is also preferable that the cell construct should have a region in which the density of the vascular cells in the central portion is $1.0 \times 10^{-4}$ cells/$\mu m^3$ or more. It is more preferable that the whole central portion of the cell construct should have the cell density described above. In this context, the phrase "have a region in which the density is $1.0 \times 10^{-4}$ cells/$\mu m^3$ or more" specifically means that a sample having the region with the density is present when 2 μm thin sliced specimens are arbitrarily prepared. The cell density is more preferably $1.0 \times 10^{-4}$ to $1.0 \times 10^{-3}$ cells/$\mu m^3$, further preferably $1.0 \times 10^{-4}$ to $2.0 \times 10^{-4}$ cells/$\mu m^3$, further preferably $1.1 \times 10^{-4}$ to $1.8 \times 10^{-4}$ cells/$\mu m^3$, further preferably $1.4 \times 10^{-4}$ to $1.8 \times 10^{-4}$ cells/$\mu m^3$. Angiogenesis can be further promoted by adopting the aforementioned range.

In this context, the density of the vascular cells in the central portion can be determined by actually counting the number of cells in a thin sliced specimen and dividing the number of cells by volume. In this context, the central portion is defined as follows: it is defined as a portion obtained by cutting out a portion corresponding to the thickness of the thin sliced specimen in the perpendicular direction in the central portion described above. In order to determine the cell density, the number of the vascular cells in the central portion can be calculated, for example, by superimposing a thin sliced specimen in which the vascular cells to be assayed have been stained and a thin sliced specimen in which cell nuclei have been stained and counting the number of overlapping cell nuclei, and the volume can be determined by determining the area of the central portion using ImageJ and multiplying the area by the thickness of the thin sliced specimen.

The cell construct for cell transplantation of the present invention encompasses those in which blood vessels have been formed using the cell construct for cell transplantation of the present invention wherein the cells are of two or more types comprising both non-vascular cells and vascular cells. Moreover, in this context, the preferable range of the "cell construct for cell transplantation of the present invention wherein the cells are of two or more types comprising both non-vascular cells and vascular cells" is as described above. Examples of a method for constructing blood vessels include a method involving bonding a cell sheet containing a vascular cell mixture to a gel material of which a piece for a blood vessel moiety has been hollowed out in a tunnel shape, and culturing the cell sheet while flowing a culture solution to the tunnel. Alternatively, vascular cells may be sandwiched between cell sheets to construct blood vessels.

(4) Method for producing cell construct for cell transplantation

The cell construct for cell transplantation of the present invention can be produced by mixing the biocompatible polymer blocks of the present invention with at least one type of cells. More specifically, the cell construct of the present invention can be produced by placing biocompatible polymer blocks (masses consisting of biocompatible polymers) and cells in an alternating manner. The production method is not particularly limited, and it is preferably a method involving forming polymer blocks and then inoculating cells thereto. Specifically, the cell construct of the present invention can be produced by incubating a mixture of the biocompatible polymer blocks and a culture solution containing the cells. For example, the cells and the biocompatible polymer blocks prepared in advance are disposed in a mosaic pattern in a vessel or in a liquid retained in a vessel. Means of this disposition is preferably use of natural aggregation, free fall, centrifugation, or stirring to promote or control the sequence formation of the mosaic pattern consisting of the cells and the biocompatible matrices.

The vessel used is preferably a vessel made of a low cell-adhesive material or a non-cell-adhesive material, and more preferably a vessel made of polystyrene, polypropylene, polyethylene, glass, polycarbonate, or polyethylene terephthalate. It is preferable that the vessel should have a flat, U-shaped, or V-shaped bottom.

From the mosaic-pattern cell construct obtained by the above described method, a cell construct having a desired size can be produced by a method, for example,

(a) fusing the separately prepared mosaic cell masses with one another, or
(b) increasing the volume thereof under a differentiation medium or growth medium.

The fusion method and the method involving an increase in volume are not particularly limited.

For example, in the step of incubating a mixture of the biocompatible polymer blocks and a culture solution containing the cells, the medium is replaced with a differentiation medium or a growth medium, whereby the volume of the cell construct can be increased. Preferably, in the step of incubating a mixture of the biocompatible polymer blocks and a culture solution containing the cells, additional biocompatible polymer blocks can be added thereto to produce a cell construct with a desired size in which the cells are uniformly present.

The above described method of fusing the separately prepared mosaic cell masses with one another is specifically a method for producing the cell construct, wherein a plurality of biocompatible polymer blocks and a plurality of cells are used, and wherein the method comprises a step of fusing a plurality of cell constructs with one another, in which one or more biocompatible polymer blocks are disposed in each of some or all of gaps formed with the plurality of cells.

The preferable ranges of the "biocompatible polymer blocks (type, size, etc.)," the "cells," the "gaps among the cells," the "obtained cell construct (size, etc.)," the "ratio between the cells and the polymer blocks," and the like according to the method for producing the cell construct of the present invention are similar to those as described in the present description.

Moreover, the thickness or diameter of each cell construct before the fusion is preferably 10 μm or more and 1 cm or less, and the thickness or diameter after the fusion is preferably 400 μm or more and 3 cm or less. In this context, the thickness or diameter of each cell construct before the fusion is more preferably 10 μm or more and 2000 μm or less, further preferably 15 μm or more and 1500 μm or less, and most preferably 20 μm or more and 1300 μm or less, and the range of the thickness or diameter after the fusion is more preferably 500 μm or more and 2 cm or less, and even more preferably 720 μm or more and 1 cm or less.

The above described method for producing a cell construct with a desired size by adding thereto additional biocompatible polymer blocks is specifically a method for producing the cell construct, comprising the steps of further adding second biocompatible polymer blocks to a cell construct and incubating them, the cell construct comprising first biocompatible polymer blocks and cells, wherein one or more of the polymer blocks are disposed in each of some or all of gaps formed by the cells. In this context, the preferable ranges of the "biocompatible polymer blocks (type, size, etc.) having biocompatibility," the "cells," the "gaps among the cells," the "obtained cell construct (size, etc.)," the "ratio between the cells and the polymer blocks," and the like are similar to those as described in the present description.

In this context, it is preferable that the cell constructs to be fused should be placed at a spacing from 0 to 50 μm, more preferably from 0 to 20 μm, further preferably from 0 to 5 μm. In the fusion of the cell constructs, the cells or substrates produced by the cells are considered to function as an adhesive by cell growth/spreading to connect the cell constructs. The adhesion between the cell constructs can be facilitated by adopting the above described range.

The range of the thickness or diameter of a cell construct obtained by the method for producing the cell construct of the present invention is preferably 400 μm or more and 3 cm or less, more preferably 500 μm or more and 2 cm or less, and even more preferably 720 μm or more and 1 cm or less.

For further adding second biocompatible polymer blocks to the cell construct and incubating them, it is preferable that the pace at which the second biocompatible polymer blocks are added should be selected appropriately according to the growth rate of the cells used. Specifically, if the second biocompatible polymer blocks are added at a fast pace, the cells are moved toward the outer region of the cell construct to reduce uniform cell distribution. If they are added at a slow pace, sites with a high ratio of the cells are formed to reduce uniform cell distribution. Thus, the pace is selected in consideration of the growth rate of the cells used.

Examples of the method for producing a cell construct comprising both non-vascular cells and vascular cells can preferably include the following production methods (a) to (c).

The production method (a) comprises a step of forming a cell construct by the above described method using non-vascular cells and then adding vascular cells and biocompatible polymer blocks thereto. In this context, the "step of adding vascular cells and biocompatible polymer blocks" encompasses both of the method involving fusing prepared mosaic cell masses with each other and the method involving increasing the volume thereof under a differentiation medium or growth medium. This method enables production of (i) a cell construct in which the non-vascular cells are present in a larger area compared with the vascular cells in the central portion of the cell construct while the vascular cells are present in a larger area compared with the non-vascular cells in the peripheral portion, (ii) a cell construct for cell transplantation in which the area of the non-vascular cells in the central portion of the cell construct is larger than the area of the non-vascular cells in the peripheral portion, and (iii) a cell construct for cell transplantation in which the area of the vascular cells in the central portion of the cell construct is smaller than the area of the vascular cells in the peripheral portion.

The production method (b) comprises a step of forming a cell construct by the above described method using vascular cells and then adding non-vascular cells and biocompatible polymer blocks thereto. In this context, the "step of adding non-vascular cells and polymer blocks" encompasses both of the method involving fusing prepared mosaic cell masses with each other and the method involving increasing the volume thereof under a differentiation medium or growth medium. This method enables production of (i) a cell construct in which the vascular cells are present in a larger area compared with the non-vascular cells in the central portion of the cell construct while the non-vascular cells are present in a larger area compared with the vascular cells in the peripheral portion, (ii) a cell construct for cell transplantation in which the area of the vascular cells in the central portion of the cell construct is larger than the area of the vascular cells in the peripheral portion, and (iii) a cell construct for cell transplantation in which the area of the non-vascular cells in the central portion of the cell construct is smaller than the area of the non-vascular cells in the peripheral portion.

The production method (c) involves substantially simultaneously using non-vascular cells and vascular cells to form a cell construct by the method described above. This method enables production of a cell construct in which neither non-vascular cells nor vascular cells are largely maldistributed at any site of the cell construct.

From the viewpoint of forming blood vessels at a transplantation site after transplantation, it is preferable to be a cell construct in which the vascular cells are present in a larger area compared with the non-vascular cells in the central portion of the cell construct while the non-vascular cells are present in a larger area compared with the vascular cells in the peripheral portion, or a cell construct for cell transplantation in which the area of the vascular cells in the central portion is larger than the area of the vascular cells in the peripheral portion. Angiogenesis can be further promoted by adopting the cell construct. The cell construct in which the number of the cells present in the central portion is larger can further promote angiogenesis.

For similar reasons, the production method comprising a step of forming a cell construct using vascular cells and then adding thereto non-vascular cells and biocompatible polymer blocks is preferable. For the production method, it is further preferable that the number of the vascular cells be increased.

(5) Intended use of cell construct for cell transplantation

The cell construct for cell transplantation of the present invention can be used for the purpose of cell transplantation at a diseased site in, for example, heart diseases such as severe heart failure and severe myocardial infarction, and cerebral ischemia/cerebral infarction. The cell construct can also be used for diseases such as diabetic kidney diseases, pancreas diseases, peripheral nerve diseases, eye diseases, or hematogenous disorder in the extremities. For example, incision, injection, or endoscopy may be used as a transplantation method. The cell construct of the present invention may be transplanted by a low invasive method such as transplantation by injection, because the size of the construct, unlike cell transplants such as cell sheets, can be decreased.

Moreover, according to the present invention, a cell transplantation method is provided. The cell transplantation method of the present invention is characterized in that it uses the above described cell construct for cell transplantation of the present invention. A preferred range of the cell construct for cell transplantation is the same as that described above.

Hereinafter, the present invention will be more specifically described. However, these examples are not intended to limit the scope of the present invention.

Examples

[Example 1] Recombinant peptide (recombinant gelatin)

CBE described below was prepared as a recombinant peptide (recombinant gelatin) (described in WO 2008-103041).

CBE3

Molecular weight: 51.6 kD
Structure: GAP$[(GXY)_{63}]_3$G
The number of amino acids: 571
The number of RGD sequences: 12
Imino acid content: 33%

Substantially 100% of amino acids are derived from the GXY repeat structures. The amino acid sequence of CBE3 does not contain serine, threonine, asparagine, tyrosine, and cysteine. CBE3 has an ERGD sequence.
Isoelectric point: 9.34, GRAVY value: -0.682, 1/IOB value: 0.323

Amino acid sequence (SEQ ID NO: 1 in the sequence listing) (same as SEQ ID NO: 3 in WO 2008/103041 except that X at the end was modified to "P")

GAP(GAPGLQGAPGLQGMPGERGAAGLPGPKGERGDAGPKGADGAPGAPGLQGM
PGERGAAGLPGPKGERGDAGPKGADGAPGKDGVRGLAGPIGPPGERGAAGLPGPK
GERGDAGPKGADGAPGKDGVRGLAGPIGPPGPAGAPGAPGLQGMPGERGAAGLPG
PKGERGDAGPKGADGAPGKDGVRGLAGPP)3G

[Example 2] Production of recombinant peptide porous body (polymer porous body)

A cylindrical cup-shaped vessel made of aluminum, having a thickness of 1 mm and a diameter of 47 mm, was prepared. When the curved surface of the cylindrical cup is defined as a side surface, the side surface is closed with 1-mm aluminum, and the bottom surface thereof (planar circular shape) was also closed with 1-mm aluminum. On the other hand, the upper surface thereof was opened. In addition, only the inside of the side surface was uniformly lined with Teflon (registered trademark) having a thickness of 1 mm, and consequently, the inner diameter of the cylindrical cup was found to be 45 mm. Hereinafter this vessel is referred to as a "cylindrical vessel."

A CBE3 aqueous solution was prepared, and the prepared CBE3 aqueous solution was then poured into the cylindrical vessel. Using a cooling shelf board, the CBE3 aqueous solution was cooled from the bottom surface in a freezer. For the cooling operation, the following conditions were prepared with regard to the temperature of the cooling shelf board, the thickness of a thermal insulation board (glass board) sandwiched between the shelf board and the cylindrical vessel, the final concentration of the CBE3 aqueous solution added, and the amount of the aqueous solution.

"A" Shelf board temperature: -40°C, the thickness of the glass board: 2.2 mm, the final concentration of the CBE3 aqueous solution: 12%, and the amount of the aqueous solution: 4 mL
"B" Shelf board temperature: -60°C, the thickness of the glass board: 2.2 mm, the final concentration of the CBE3 aqueous solution: 7.5%, and the amount of the aqueous solution: 4 mL
"C" Shelf board temperature: -40°C, the thickness of the glass board: 2.2 mm, the final concentration of the CBE3 aqueous solution: 4.0%, and the amount of the aqueous solution: 4 mL

The thus obtained frozen CBE3 blocks were freeze-dried to obtain CBE3 porous bodies.

[Comparative Example 1] production of porous body of simply frozen recombinant peptide

2000 mg of CBE3 was dissolved in 18 mL of ultrapure water at 50°C, so as to produce 20 mL of a CBE3 solution having a final concentration of 10%. This CBE3 solution was thinly spread to produce a thin planar gel with a thickness of approximately 4 mm. Regarding a vessel, a silicon frame (approximately 5 cm × 10 cm) was attached to a white plate, and the silicon frame was strongly pressed on the white plate so as not to generate air gaps. Then, the aforementioned CBE3 solution (50°C) was poured into the frame. After the solution had poured into the frame, the temperature was decreased to 4°C, and the solution was gelatinized for approximately 1 hour. After confirmation of the gelatinization, the temperature was further decreased to -80°C, and the gel was frozen for 3 hours. After the gel had been frozen, it was freeze-dried using a freeze dryer (EYELA, FDU-1000). The thus obtained freeze-dried product was a porous body, and the mean pore size thereof was 57.35 μm. Hereinafter, this porous body is referred to as a "simply frozen porous body."

[Example 3] Evaluation of pore size and space occupation percentage of recombinant peptide porous body

With regard to the CBE3 porous body obtained in Example 2 and the simply frozen porous body obtained in Comparative Example 1, the pore size and space occupation percentage of each porous body were evaluated. The obtained porous bodies were each subjected to thermal crosslinking at 160°C for 20 hours, so as to insolubilize them. Thereafter, the porous bodies were swollen with a normal saline for a sufficient period of time. Thereafter, a frozen tissue section was prepared using a microtome, and then, a HE (hematoxylin-eosin)-stained specimen was produced. From the specimen, a section image with a real scale of 1.5 mm was prepared, and the area of each pore was measured. Then, the diameter of a circle, which was obtained when the measured area was calculated relative to a circle, was calculated, and the obtained circle diameter was defined as a pore size. A mean value of 20 or more pores was defined as a mean pore size. As a result, in the case of "A," the mean pore size was found to be 66.39 μm, in the case of "B," it was found to be 63.17 μm, and in the case of "C," it was found to be 56.36 μm.

Using the two-dimensional section image as used above, the space occupation percentage was obtained by dividing the area of pores having a certain pore size by the total area, based on the calculated pore sizes. As a result, in the case of the CBE3 porous body obtained in Example 2, the space occupation percentage of pores with a pore size of 20 μm to 200 μm was 100% in the case of "A," 99.9% in the case of "B," and 99.9% in the case of "C." In addition, the space occupation percentage of pores with a pore size of 30 μm to 150 μm was 94.2% in the case of "A," 97.9% in the case of "B," and 99.3% in the case of "C."

On the other hand, in the case of the simply frozen porous body obtained in Comparative Example 1, pores largely varied in terms of pore size. Thus, sites in which pores with a large size were gathered, and sites in which pores with a small size were gathered, were present. In the sites in which pores with a large size were gathered, the space occupation percentage of pores with a pore size of 20 μm to 200 μm was 74.3%, and the space occupation percentage of pores with a pore size of 30 μm to 150 μm was 55.8%. In the sites in which pores with a small size were gathered, the space occupation percentage of pores with a pore size of 20 μm to 200 μm was 82.8%, and the space occupation percentage of pores with a pore size of 30 μm to 150 μm was 57.2%. Since the sites in which pores with a large pore size were gathered, and the site in which pores with a small pore size were gathered, were present half and half, the space occupation percentage of pores with a pore size of 20 μm to 200 μm became 78.6%, and the space occupation percentage of pores with a pore size of 30 μm to 150 μm became 56.5% (Figure 6).

[Example 4] Measurement of porosity of recombinant peptide porous body

The porosity of the CBE3 porous body obtained in Example 2 was measured. Upon the measurement of the porosity, the apparent density (p) and the true density (pc) were measured, and the porosity ($P = 1-\rho/\rho c$ (%)) was then obtained. The apparent density (p) of the CBE3 porous body was calculated based on the dry mass and the volume. The true density (pc) was obtained by a specific gravity bottle method using a Hubbard bottle. As a result of the measurement of samples (the number of samples (N) = 4), it became clear that the porous body in the case of "C" had an apparent density of 0.05 g/$cm^3$, a true density of 1.23 g/$cm^3$, and a porosity of 96% (CV value: 8%). Moreover, it was also found that the porous bodies in the cases of "A" and "B" had a porosity of 87% (CV value: 10%) and 92% (CV value: 7%), respectively.

[Example 5] Production of recombinant peptide block (crushing and crosslinking of porous body)

The CBE3 porous bodies "A," "B" and "C," obtained in Example 2, were crushed using New Power Mill (Osaka Chemical Co.., Ltd., New Power Mill PM-2005). Such crushing was carried out at a maximum engine speed for 1 minute × 5 times, namely, for 5 minutes in total. Using a sieve made of stainless steel, the crushed product was divided in terms of size, so as to obtain CBE3 blocks with sizes of 25 to 53 μm, 53 to 106 μm, and 106 μm to 180 μm. Thereafter, the

blocks were subjected to thermal crosslinking at 160°C under reduced pressure (9 types of crosslinking times, namely, 24 hours, 48 hours, 56 hours, 60 hours, 72 hours, 84 hours, 96 hours, 120 hours, and 288 hours were applied), thereby obtaining samples. Hereinafter, the porous body "A" with a size of 53 to 106 μm is referred to as "12% middle," the porous body "B" with a size of 25 to 53 μm is referred to as "7.5% small," the porous body B" with a size of 53 to 106 μm is referred to as "7.5% middle," the porous body "B" with a size of 106 to 180 μm is referred to as "7.5% large," and the porous body "C" with a size of 53 to 106 μm is referred to as"4% middle."

[Comparative Example 2] Production of GA (glutaraldehyde)-crosslinked μ block of recombinant peptide

As a comparative example described in Patent Literature 5, in which glutaraldehyde is used, using the recombinant peptide CBE3 as a base material, an amorphous GA-crosslinked μ block was produced. 1000 mg of CBE3 was dissolved in 9448 μL of ultrapure water, and 152 μL of 1N HCl was then added to the obtained solution. Thereafter, 400 μL of 25% glutaraldehyde was added to the mixed solution to a final concentration of 1.0%. The obtained mixture was reacted at 50°C for 3 hours to produce crosslinked gel. This crosslinked gel was immersed in 1 L of 0.2 M glycine solution, and it was then shaken at 40°C for 2 hours. Thereafter, the crosslinked gel was washed by shaking in 5 L of ultrapure water for 1 hour. After that, the ultrapure water was replaced with a fresh one, and washing was then repeated for 1 hour. Thus, washing was carried out 6 times in total. The thus washed crosslinked gel was frozen at -80°C for 5 hours, and was then freeze-dried using a freeze dryer (EYELA, FDU-1000). The obtained freeze-dried product was crushed with New Power Mill (Osaka Chemical Co.., Ltd., New Power Mill PM-2005). Crushing was carried out at a maximum engine speed for 1 minute × 5 times, namely, for 5 minutes in total. Using a sieve made of stainless steel, the crushed product was divided in terms of size, so as to obtain CBE3 · GA-crosslinked μ blocks with a size of 25 to 53 μm.

[Comparative Example 3] Production of comparative recombinant peptide block

As a comparative example, a comparative recombinant peptide block that can be produced by a step of not involving glutaraldehyde, which can be estimated from the prior art (Patent Literature 5), was produced as follows. Using the recombinant peptide CBE3 as a base material, a block was produced. The simply frozen porous body obtained in Comparative Example 1 was crushed with New Power Mill (Osaka Chemical Co.., Ltd., New Power Mill PM-2005). Crushing was carried out at a maximum engine speed for 1 minute × 5 times, namely, for 5 minutes in total. Using a sieve made of stainless steel, the crushed product was divided in terms of size, so as to obtain CBE3 blocks with a size of 25 to 53 μm. The obtained CBE3 blocks were placed in an oven of 160°C, and were thermally crosslinked for 72 hours.

[Example 6] Measurement of tap density of recombinant peptide blocks

The tap density is a value indicating how many blocks can be filled in a certain volume. As the value of the tap density decreases, it indicates that the blocks cannot be densely filled, namely, the structure of the blocks is complicated. The tap density was measured as follows. First, a funnel, to the tip of which a cap (a cylindrical cap with a diameter of 6 mm and a length of 21.8 mm; volume: 0.616 $cm^3$) was attached, was prepared, and the mass of a cap alone was measured. Thereafter, the cap was fixed to the funnel, and blocks were then supplied into the cap through the funnel, so that the blocks were accumulated in the cap. After a sufficient amount of blocks had been placed in the cap, the cap portion was slammed against a hard stuff such as a desk 200 times. Thereafter, the funnel was removed from the cap, and the blocks were then leveled using a spatula. The mass of one level cap of blocks was measured. The mass of the blocks alone was calculated based on the difference between the mass of the cap alone and the mass of the one level cap of blocks. The mass of the blocks alone was divided by the volume of the cap to obtain a tap density.

As a result, the tap density of the blocks of Comparative Example 3 was found to be 524 mg/$cm^3$.

On the other hand, in the case of the blocks of Example 5, the tap density of "12% middle" was 372 mg/$cm^3$, the tap density of "7.5% small" was 213 mg/$cm^3$, the tap density of "7.5% middle" was 189 mg/$cm^3$, the tap density of "7.5% large" was 163 mg/$cm^3$, and the tap density of "4% middle" was 98 mg/$cm^3$. It was found that the tap density of the blocks of Example 5 was smaller than the tap density of the blocks of Comparative Example 3, due to the complexity of the structure thereof.

[Example 7] Calculation of "square root of the area / boundary length" in two-dimensional section image of recombinant peptide block

As an indicator showing the complexity of a block, the relationship between the "square root of the area" of a block and the "boundary length" thereof was obtained. That is to say, it can be said that as the value of the "square root of the area / boundary length" of a block decreases, the block is more complicated. This value was calculated using image analysis software. First, an image, in which the shape of a block could be seen, was prepared. Specifically, in the present example, from a group of blocks that had been fully swollen with water, frozen sections were prepared using a microtome, and the sections were then stained with HE (hematoxylin-eosin) to produce specimens. In a case where cells and the like, other than blocks, were present, only a formulation was extracted by an automatic selection tool of the photoshop, so that only the blocks were allowed to be present on the image. The area of a block and the boundary length thereof were obtained from the image, using ImageJ, and the value of the "square root of the area / boundary length" was then calculated. Blocks with a size of 10 μm or less were excluded.

As a result, the value of the blocks of Comparative Example 3 was 0.139.

On the other hand, in the case of the blocks of Example 5, the value of "12% middle" was 0.112, the value of "7.5% small" was 0.083, the value of "7.5% middle" was 0.082, the value of "7.5% large" was 0.071, and the value of "4% middle" was 0.061. It was found that the value of the "square root of the area / boundary length" of the blocks of Example 5 was smaller than the value of the blocks of Comparative Example 3, due to the complexity of the structure thereof. It was also found that the value of the "square root of the area / boundary length" correlates with the tap density.

[Example 8] Measurement of degree of cross-linkage of recombinant peptide block

The degree of cross-linkage (the number of cross-linkages per molecule) of the blocks, which had been crosslinked in Example 5 and Comparative Example 3, was calculated. For the measurement of the degree of cross-linkage, a TNBS (2,4,6-trinitrobenzenesulfonic acid) method was applied.

< Preparation of sample >

Approximately 10 mg of a sample, 1 mL of a 4% $NaHCO_3$ aqueous solution, and 2 mL of a 1% TNBS aqueous solution were added to a glass vial, and the obtained mixture was then shaken at 37°C for 3 hours. Thereafter, 10 mL of 37% hydrochloric acid and 5 mL of pure water were added to the reaction solution, and the obtained mixture was then left at rest at 37°C for 16 or more hours. The resultant was used as a sample.

< Preparation of blank >

Approximately 10 mg of a sample, 1 mL of a 4% $NaHCO_3$ aqueous solution, and 2 mL of a 1% TNBS aqueous solution were added to a glass vial, and immediately after the addition, 3 mL of 37% hydrochloric acid was added thereto, and the obtained mixture was shaken at 37°C for 3 hours. Thereafter, 7 mL of 37% hydrochloric acid and 5 mL of pure water were added to the reaction solution, and the obtained mixture was then left at rest at 37°C for 16 or more hours. The resultant was used as a blank.

The absorbance (345 nm) of each of the sample and the blank, which had been 10 times diluted with pure water, was measured, and the degree of cross-linkage (the number of cross-linkages per molecule) was then calculated according to the following (Formula 1) and (Formula 2).

$$\text{(Formula 1)}\ (As\text{-}Ab)/14600 \times V/w$$

(Formula 1) shows the amount (equimolar amount) of lysine per g of the recombinant peptide.

(In the above formula, As represents the absorbance of the sample, Ab represents the absorbance of the blank, V represents the amount of the reaction solution (g), and w represents the mass (mg) of the recombinant peptide.)

$$\text{(Formula 2)}\ 1\text{- (sample (Formula 1) / uncrosslinked polymer (Formula 1))} \times 34$$

(Formula 2) shows the number of cross-linkages per molecule.

As a result, with regard to the blocks of Example 5, the degree of cross-linkage of "12% middle" was 12 after crosslinking for 48 hours. The degree of cross-linkage of each of "7.5% small," "7.5% middle" and "7.5% large" was 8 after crosslinking for 24 hours. The degree of cross-linkage of "4% middle" was 9 after crosslinking for 48 hours. In addition, the degree of cross-linkage of "7.5% middle" was 22, when it was crosslinked for 288 hours. On the other hand, the degree of cross-linkage of the blocks of Comparative Example 3 was 16 after crosslinking for 72 hours.

[Example 9] Measurement of water absorption percentages of recombinant peptide blocks

The water absorption percentages of the blocks produced in Example 5 and Comparative Example 3 were calculated.

Approximately 15 mg of blocks were filled into a bag made of nylon mesh with a size of 3 cm × 3 cm at 25°C, and the blocks were then swollen in ion exchange water for 2 hours. Thereafter the resulting blocks were air-dried for 10 minutes. The mass thereof was measured at individual stages, and then, the water absorption percentage was obtained according to the following (Formula 3).

(Formula 3)

$$\text{Water absorption percentage} = (w2 - w1 - w0) / w0$$

(In the above formula, w0 represents the mass of the material before water absorption, w1 represents the mass of the empty bag after water absorption, and w2 represents the mass of the entire bag that contains the material after water absorption.)

As a result, with regard to the blocks of Example 5, the water absorption percentage of "12% middle" was 304%, the water absorption percentage of "7.5% small" was 819%, the water absorption percentage of "7.5% middle" was 892%, the water absorption percentage of "7.5% large" was 892%, and the water absorption percentage of "4% middle" was 901%. On the other hand, the water absorption percentage of the blocks of Comparative Example 3 was 280%.

[Example 10] Preparation of mosaic cell mass (hMSC) using recombinant peptide blocks

Human bone marrow-derived mesenchymal stem cells (hMSCs) were adjusted to a cell density of 100,000 cells/mL with a growth medium (Takara Bio Inc.; MSCGM BulletKit™). After addition of the CBE3 blocks prepared in Example 5 to the cells to a concentration of 0.1 mg/mL, 200 μL of the mixture was inoculated on a Sumilon Celltight X96U plate (Sumitomo Bakelite Co., Ltd., U-shaped bottom), was then centrifuged (600 g, 5 minutes) with a tabletop plate centrifuge, and was then left at rest for 24 hours, so as to prepare a spherical mosaic cell mass with a diameter of approximately 1 mm that consisted of the CBE3 blocks and the hMSC cells (0.001μg of blocks per cell). Since the present mosaic cell mass was prepared in a U-shaped plate, it was spherical. For the blocks "12% middle," "7.5% small," "7.5% middle," "7.5% large," and "4% middle", the mosaic cell mass could be prepared in the same manner as described above.

[Comparative Example 4] Preparation of mosaic cell mass (hMSC) using comparative recombinant peptide blocks

Human bone marrow-derived mesenchymal stem cells (hMSCs) were adjusted to a cell density of 100,000 cells/mL with a growth medium (Takara Bio Inc.; MSCGM BulletKit™). After addition of the CBE3 blocks prepared in Comparative Example 3 to the cells to a concentration of 0.1 mg/mL, 200 μL of the mixture was inoculated on a Sumilon Celltight X96U plate (Sumitomo Bakelite Co., Ltd., U-shaped bottom), was then centrifuged (600 g, 5 minutes) with a tabletop plate centrifuge, and was then left at rest for 24 hours, so as to prepare a spherical mosaic cell mass with a diameter of approximately 1 mm that consisted of the CBE3 blocks and the hMSC cells (0.001μg of blocks per cell). Since the present mosaic cell mass was prepared in a U-shaped plate, it was spherical.

[Comparative Example 5] Preparation of mosaic cell mass (hMSC) using recombinant peptide GA-crosslinked μ blocks

A mosaic cell mass comprising glutaraldehyde, which was to be used as a comparative example, was prepared as follows. Human bone marrow-derived mesenchymal stem cells (hMSCs) were adjusted to a cell density of 100,000 cells/mL with a growth medium (Takara Bio Inc.; MSCGM BulletKit™). After addition of the GA-crosslinked μ blocks prepared in Comparative Example 2 to the cells to a concentration of 0.1 mg/mL, 200 μL of the mixture was inoculated on a Sumilon Celltight X96U plate (Sumitomo Bakelite Co., Ltd., U-shaped bottom), was then centrifuged (600 g, 5 minutes) with a tabletop plate centrifuge, and was then left at rest for 24 hours, so as to prepare a spherical mosaic cell mass with a diameter of approximately 1 mm that consisted of the GA-crosslinked μ blocks and the hMSC cells (0.001μg of blocks per cell). Since the present mosaic cell mass was prepared in a U-shaped plate, it was spherical.

[Example 11] Preparation of mosaic cell mass (hMSC + hECFC) using recombinant peptide blocks

Human endothelial colony forming cells (hECFCs) were adjusted to a cell density of 100,000 cells/mL with a growth medium (Lonza: EGM-2 + ECFC serum supplement). After addition of the CBE3 blocks prepared in Example 5 to the cells to a concentration of 0.05 mg/mL, 200 μL of the mixture was inoculated on a Sumilon Celltight X96U plate (Sumitomo Bakelite Co., Ltd., U-shaped bottom), was then centrifuged (600 g, 5 minutes) with a tabletop plate centrifuge, and was then left at rest for 24 hours, so as to prepare a flat mosaic cell mass consisting of the ECFCs and the CBE3 blocks. Thereafter, the medium was removed, and human bone marrow-derived mesenchymal stem cells (hMSCs) were adjusted to a cell density of 100,000 cells/mL with a growth medium (Takara Bio Inc.; MSCGM BulletKit™). After addition of the CBE3 blocks prepared in Example 5 to the cells to a concentration of 0.1 mg/mL, 200 μL of the mixture comprising hECFC mosaic cell mass was inoculated on a Sumilon Celltight X96U plate (Sumitomo Bakelite Co., Ltd., U-shaped bottom), was then centrifuged (600 g, 5 minutes) with a tabletop plate centrifuge, and was then left at rest for 24 hours, so as to prepare a spherical mosaic cell mass with a diameter of approximately 1 mm that consisted of the hMSCs, the hECFCs and the CBE3 blocks. For the blocks "12% middle," "7.5% small," "7.5% middle," "7.5% large," and "4% middle", the mosaic cell mass could be prepared in the same manner as described above.

[Example 12] Fusion of mosaic cell masses (hMSC) using recombinant peptide blocks

Five of the mosaic cell masses (comprising the CBE3 blocks of the present invention) on Day 2 after they had been prepared in Example 10 were arranged in a Sumilon Celltight X96U plate, and they were then cultured for 24 hours. As a result, it was revealed that the cells placed on the periphery of each mosaic cell mass bound the mosaic cell masses to one another, whereby the mosaic cell masses were naturally fused. For the blocks "12% middle," "7.5% small," "7.5% middle," "7.5% large," and "4% middle", the fusion of mosaic cell mass could be carried out in the same manner as described above.

[Example 13] Fusion of mosaic cell masses (hMSC + ECFC) using recombinant peptide blocks

Five of the mosaic cell masses (comprising the CBE3 blocks of the present invention) on Day 2 after they had been prepared in Example 11 were arranged in a Sumilon Celltight X96U plate, and they were then cultured for 24 hours. As a result, it was revealed that the cells placed on the periphery of each mosaic cell mass bound the mosaic cell masses to one another, whereby the mosaic cell masses were naturally fused. For the blocks "12% middle," "7.5% small," "7.5% middle," "7.5% large," and "4% middle", the fusion of mosaic cell mass could be carried out in the same manner as described above.

[Comparative Example 6] Fusion of mosaic cell masses (hMSC) using comparative recombinant peptide blocks

Five of the mosaic cell masses (derived from the comparative CBE3 blocks) on Day 2 after they had been prepared in Comparative Example 4 were arranged in a Sumilon Celltight X96U plate, and they were then cultured for 24 hours. As a result, it was revealed that the cells placed on the periphery of each mosaic cell mass bound the mosaic cell masses to one another, whereby the mosaic cell masses were naturally fused.

[Comparative Example 7] Fusion of mosaic cell masses (hMSC) using recombinant peptide GA-crosslinked μ blocks

Five of the mosaic cell masses (derived from the GA-crosslinked μ blocks) on Day 2 after they had been prepared in Comparative Example 5 were arranged in a Sumilon Celltight X96U plate, and they were then cultured for 24 hours. As a result, it was revealed that the cells placed on the periphery of each mosaic cell mass bound the mosaic cell masses to one another, whereby the mosaic cell masses were naturally fused.

[Example 14] *In vitro* ATP assay

The amount of ATP (adenosine triphosphate) produced/retained by the cells in each mosaic cell mass was determined. ATP is known as an energy source for general organisms. The active metabolic state and activity state of cells can be understood by determining the amount of ATP synthesized/retained. CellTiter-Glo (Promega) was used in the measurement. Using the CellTiter-Glo, the amount of ATP in each mosaic cell mass was quantified for the mosaic cell masses prepared in Examples 10, Comparative Example 4, and Comparative Example, all of which were of Day 7. As a result, it was found that the amount of ATP was smaller in the mosaic cell mass prepared using the comparative CBE3 blocks than in the mosaic cell mass prepared using the GA-crosslinked μ blocks. On the other hand, it was found that the amount of ATP was larger in the mosaic cell mass prepared using the CBE3 blocks of the present invention than in the mosaic cell mass prepared using GA-crosslinked μ blocks. In all cases of "12% middle," "7.5% small," "7.5% middle," "7.5% large," and "4% middle," the results were obtained that a larger amount of ATP was generated in the mosaic cell mass prepared using the CBE3 blocks of the present invention than in the mosaic cell mass prepared using the GA-crosslinked μ blocks (Figure 1). In other words, it became clear that the survival state of cells in the mosaic cell mass prepared using the CBE3 blocks of the present invention having a complicated structure was better than that in the other mosaic cell masses.

[Example 15] Production of enormous mosaic cell mass using recombinant peptide blocks

It is possible to prepare an enormous mosaic cell mass by fusing mosaic cell masses with a size of 1 mm with one another, as described in Example 12. However, the operations can be simplified, if such an enormous mosaic cell mass can be prepared at one time. In the present example, a 9-cm petri dish of Sumilon Celltight was processed such that cells could not adhere thereto, and 50 mL of 1.5% agarose (Agarose S) solution in a growth medium (Takara Bio Inc.; MSCGM BulletKit™) was then placed in the petri dish. At that time, a 1-cm square rod-shaped silicon was immobilized such that approximately 5 mm of the silicon was immersed in the agarose solution, and after the agarose had been consolidated, the silicon was removed, so that a vessel in which a 1-cm square void was formed in the agarose was prepared. To the vessel, an appropriate amount of growth medium was placed, and it was then preserved while paying attention that the gel was not dried. Thereafter, the medium was removed, and a suspension consisting of 16 mg of the blocks "7.5% middle," which was typical blocks among the blocks prepared in Example 5, and human bone marrow-derived mesenchymal stem cells (hMSCs) (2,500,000 cells), was placed in the void. Thereafter, 25 mL of a growth medium was gently added to the suspension. The obtained mixture was cultured for 1 day, and a 1-cm square enormous mosaic cell mass with a thickness of 2 to 3 mm could be prepared. The thus prepared cell mass was transferred into a 9-cm petri dish of Sumilon Celltight, in which 25 mL of a growth medium had been placed, and the obtained mixture was further cultured for 2 days. Thereafter, the culture was transferred into a spinner flask, in which 25 mL of a growth medium had been placed, and the obtained mixture was subjected to shaking culture. Seven days after the culture, a HE-stained specimen of the section of the mosaic cell mass was prepared. As a result, it was confirmed that internal cells still survived even 7 days after the culture. Thus, it became clear that an enormous mosaic cell mass could be produced by mixing cells with blocks, supplying the mixture into a certain mold, and then culturing it. Moreover, this method can be applied using any of GA-crosslinked μ blocks, comparative blocks, and the blocks of the present invention, and thus, it does not depend on the type of blocks.

[Example 16] Transplantation of mosaic cell mass using recombinant peptide blocks

Four- to six-week-old male NOD/SCID mice (Charles River Laboratories Japan, Inc.) were used. The abdominal hair of each mouse was removed under anesthesia. The upper abdominal region was subcutaneously slit up, and scissors were inserted through the slit to take off the skin from the muscle. Then, the mosaic cell masses prepared in Example 12, Example 13, Comparative Example 6, and Comparative Example 7 were each scooped with tweezers and were each subcutaneously transplanted in the later abdominal region 1.5 cm below the slit, and the slit in the skin was sutured.

[Example 17] Collection of mosaic cell mass using recombinant peptide blocks

Anatomy was performed 1 week or 2 weeks after transplantation. The skin in the abdominal region was taken off, and the skin to which each mosaic cell mass was attached was cut into a square of approximately 1 $cm^2$ in size. In the case where the mosaic cell mass was also attached to the muscle in the abdominal region, the mosaic cell mass was collected together with the muscle.

[Example 18] Analysis of specimen

A tissue slice was prepared for the skin slice to which the mosaic cell mass was attached and the mosaic cell masses before transplantation. The skin was dipped in 4% paraformaldehyde, and formalin fixation was performed. Then, the resulting product was embedded in paraffin to prepare a tissue slice of the skin containing each mosaic cell mass. The slice was stained with HE (hematoxylin-eosin).

HE specimens 2 weeks after transplantation of the "7.5% middle" of Example 12, Comparative Example 6, and Comparative Example 7, are shown in Figure 2. The survival rate shown in Figure 2 indicates the ratio of the number of living cells to the total number of cells (the number of living cells + the number of dead cells).

It was found that the number of living cells in the mosaic cell mass comprising the blocks of Comparative Example 6 (Figure 2B; the number of living cells: 37; survival rate: 45%) is smaller than the number of living cells in the mosaic cell mass comprising the GA-crosslinked μ blocks of Comparative Example 7 (Figure 2A; the number of living cells: 100; survival rate: 80%). On the other hand, it was found that the number of living cells in the mosaic cell mass comprising the blocks of the present invention ("7.5% middle") of Example 12 (Figure 2C; the number of living cells: 116; survival rate: 84%) is larger than the number of living cells in the mosaic cell mass comprising the blocks of Comparative Example 6 (Figure 2B; the number of living cells : 37; survival rate: 45%), and that the cells in the mosaic cell mass comprising the present blocks "7.5% middle" highly survived. These results correspond to the results of the *in vitro* assay performed in Example 14, and it was found that the survival rate of the transplanted cells can be increased by adopting the blocks of the present invention.

Moreover, the survival states of the transplanted cells among the block groups of the present invention are shown in Figure 3. The survival rate shown in Figure 3 indicates the ratio of the number of living cells to the total number of cells (the number of living cells + the number of dead cells).

The survival rate of the blocks "7.5% large" with a size of 106 to 180 μm was 57%, the survival rate of "7.5% small" was 62%, the survival rate of "7.5% middle" was 84%, and the survival rate of "4% middle" was 82%. That is to say, it was found that, among the block groups of the present invention, the survival rate of cells becomes further higher with the blocks "7.5% small," "7.5% middle" and "4% middle," than with the blocks"7.5% large" with a size of 106 to 180 μm (Figure 3). It was also found that the blocks "7.5% middle" and "4% middle" provides higher survival rate of the transplanted cells than the blocks "7.5% small" do, and thus that these blocks provide the best transplantation results (Figure 3). That is, it was further found that it is important for a polymer block to have a structure such that the tap density thereof or the value of the square root of the cross-sectional area / boundary length of the polymer block in a two-dimensional section image can be within the predetermined range, and that, among others, the survival state of the transplanted cells is improved in the order of "53 to 106 μm" > "25 to 53 μm" > "106 to 180 μm."

Moreover, a HE specimen two weeks after transplantation in a case where the mosaic cell mass of Example 12 comprising the blocks of the present invention with the concerned size of 53 to 106 μm has been transplanted is shown in Figure 4. As a result of counting the number of blood vessels shown in Figure 4, it was found to be 63 blood vessels/mm$^2$. As shown in Figure 4, it was also found that blood vessels are drawn into the mosaic cell mass.

Furthermore, with regard to the cell mass comprising representative blocks "7.5% middle" among the cell masses in Example 13, a HE specimen two weeks after transplantation in a case where the mosaic cell mass comprising vascular cells has been transplanted is shown in Figure 5. As a result of counting the number of blood vessels shown in Figure 5, it was found to be 180 blood vessels/mm$^2$. As shown in Figure 5, it has been clarified that in comparison with the case where the cell mass of Example 12 was transplanted, when the mosaic cell mass comprising vascular cells of Example 13 was transplanted, much more blood vessels were formed in the mosaic cell mass.

[Example 19] Calculation of ratio and concentration of ECFC in hMSC + hECFC mosaic cell mass

Among the cell masses of Example 11, the mosaic cell mass comprising representative blocks "7.5% middle" was immunostained with an anti-CD31 antibody (EPT, Anti CD31/PECAM-1) for hECFC staining using a kit using DAB color development (Dako LSAB2 kit, Universal, K0673 Dako LSAB2 kit/HRP (DAB), for use with both rabbit and mouse primary antibodies). The ratio of the area of hECFCs (vascular cells) in the central portion was determined for the aforementioned mosaic cell mass comprising representative blocks "7.5% middle," using the image processing software ImageJ described above and the staining method using an anti-CD31 antibody. In this context, the "central portion" is as defined above.

As a result, the ratio of the area of hECFCs (vascular cells) in the central portion of the mosaic cell mass comprising the representative blocks "7.5% middle" of Example 11 was 99%.

Furthermore, the density of the hECFC cells present in the central portion was calculated for the mosaic cell mass comprising the representative blocks "7.5% middle" of Example 11 by superimposing the anti-CD31 antibody staining image and the HE staining (hematoxylin-eosin staining) image. The density of the vascular cells in the central portion can be determined by actually counting the number of cells in a thin sliced specimen and dividing the number of cells by volume. First, these two images were superimposed using Photoshop, and the number of anti-CD31 antibody-stained cell nuclei overlapping with HE stained cell nuclei was counted to calculate the number of cells. Meanwhile, the volume was determined by obtaining the area of the central portion using ImageJ and multiplying the obtained area by 2 μm as the thickness of the thin sliced specimen.

As a result, the number of the hECFC cells (vascular cells) in the central portion of the mosaic cell mass comprising the representative blocks "7.5% middle" of Example 11 was $2.58 \times 10^{-4}$ cells/μm$^3$.

[Example 20] Production of recombinant peptide porous body (polymer porous body)

A cylindrical cup-shaped vessel made of aluminum, having a thickness of 1 mm and a diameter of 47 mm, was prepared. When the curved surface of the cylindrical cup is defined as a side surface, the side surface is closed with 1-mm aluminum, and the bottom surface thereof (planar circular shape) was also closed with 1-mm aluminum. On the other hand, the upper surface thereof was opened. In addition, only the inside of the side surface was uniformly lined with Teflon (registered trademark), and consequently, the inner diameter of the cylindrical cup was found to be 45 mm. Hereinafter this vessel is referred to as a "cylindrical vessel."

A recombinant peptide aqueous solution, in which the final concentration of CBE3 was 7.5% by mass, was prepared. The prepared recombinant peptide aqueous solution was poured into a cylindrical vessel. Using a cooling shelf board, the recombinant peptide aqueous solution was cooled from the bottom surface in a freezer. During the cooling operation, the temperature of the cooling shelf board, the thickness of a thermal insulation board (glass board) sandwiched between the shelf board and the cylindrical vessel, and the amount of the added recombinant peptide aqueous solution were changed, so that the cooling process regarding liquid temperature was also changed. The shelf board temperature was set at -40°C, -60°C and -80°C, the thickness of the glass board was set at 0.7 mm, 1.1 mm and 2.2 mm, and the amount of the recombinant peptide aqueous solution was set at 4 mL, 12 mL and 16 mL. The experiment was carried out with combinations of these conditions.

Moreover, since each aqueous solution was cooled from the bottom surface, the water surface temperature of the central portion of the circle was most hardly cooled. Accordingly, since the aforementioned portion had the highest liquid temperature in the solution, the liquid temperature in that portion was measured (hereinafter, the liquid temperature in that portion is referred to as the "highest internal liquid temperature").

As a result, when the shelf board temperature was -40°C and the thickness of the glass board was 2.2 mm, temperature rise did not begin until the highest internal liquid temperature would become -9.2°C, and the highest internal liquid temperature was "the melting point of a solvent - 3°C" or lower in an unfrozen state (Figure 8). After this state, temperature rise began at -9.2°C, and it is found that the heat of solidification was generated (Figure 8). Moreover, it could also be confirmed that ice formation actually began at that timing. Thereafter, the temperature has moved around 0°C for a certain period of time. During this period, a mixture of water and ice was present. Finally, temperature drop began again at 0°C. At this time, the liquid portion disappeared, and it became ice (Figure 8). The measured temperature became the solid temperature in the ice. That is, the temperature has not been liquid temperature any more. Thus, if the highest internal liquid temperature is observed in the moment at which the heat of solidification is generated, it is found whether the liquid is frozen after the highest internal liquid temperature has passed "the melting point of a solvent - 3°C" in an unfrozen state.

Six types of combinations of highest internal liquid temperatures in an unfrozen state at the moment in which the heat of solidification was generated were measured. The results are as follows.

A. The highest internal liquid temperature was -9.2°C under conditions of a shelf board temperature of -40°C, a glass board thickness of 2.2 mm, and a liquid amount of 4 mL.
B. The highest internal liquid temperature was -8.3°C under conditions of a shelf board temperature of -40°C, a glass board thickness of 1.1 mm, and a liquid amount of 4 mL.
C. The highest internal liquid temperature was -2.2°C under conditions of a shelf board temperature of -40°C, a glass board thickness of 0.7 mm, and a liquid amount of 4 mL.
D. The highest internal liquid temperature was -7.2°C under conditions of a shelf board temperature of -60°C, a glass board thickness of 2.2 mm, and a liquid amount of 4 mL.

It is to be noted that the above D corresponds to "B" in Example 2.

E. The highest internal liquid temperature was -3.9°C under conditions of a shelf board temperature of -80°C, a glass board thickness of 2.2 mm, and a liquid amount of 4 mL.
F. The highest internal liquid temperature was -3.1°C under conditions of a shelf board temperature of -80°C, a glass board thickness of 1.1 mm, and a liquid amount of 4 mL.
G. The highest internal liquid temperature was 5.8°C under conditions of a shelf board temperature of -80°C, a glass board thickness of 0.7 mm, and a liquid amount of 4 mL.
H. The highest internal liquid temperature was -6.5°C under conditions of a shelf board temperature of -40°C, a glass board thickness of 2.2 mm, and a liquid amount of 12 mL.
I. The highest internal liquid temperature was -2.4°C under conditions of a shelf board temperature of -40°C, a glass board thickness of 2.2 mm, and a liquid amount of 16 mL.

From these results, A, B, D, E, F, and H are production methods comprising a freezing step in which the highest internal liquid temperature becomes "the melting point of a solvent - 3°C" or lower in an unfrozen state. (A frozen recombinant peptide block, regarding which the highest internal liquid temperature ≤ "the melting point of a solvent - 3°C.")

Moreover, C, G, and I are production methods comprising a freezing step in which the highest internal liquid temperature does not become "the melting point of a solvent - 3°C" or lower in an unfrozen state. (A frozen recombinant peptide block, regarding which the highest internal liquid temperature > "the melting point of a solvent - 3°C.")

The thus obtained frozen recombinant peptide blocks were freeze-dried to obtain CBE3 porous bodies. The CBE3 porous bodies according to A, B, D, E, F, and H are referred to as "CBE3 porous bodies, regarding which the highest internal liquid temperature ≤ "the melting point of a solvent - 3°C."" On the other hand, the "CBE3 porous bodies according to C, G, and I are referred to as "CBE3 porous bodies, regarding which the highest internal liquid temperature > "the melting point of a solvent - 3°C.""

[Example 21]

The CBE3 porous bodies obtained in Example 20 were evaluated in terms of the pore size of each porous body and the shape of pores. The obtained porous bodies were each subjected to thermal crosslinking at 160°C for 20 hours to insolubilize them. Thereafter, the resulting porous bodies were swollen with a normal saline for a sufficient period of time. Subsequently, frozen tissue sections were prepared using a microtome, and HE (hematoxylin-eosin)-stained specimens were then produced.

The images of the central portions of the obtained specimens are shown in Figure 7 (both in the case of the highest internal liquid temperature > "the melting point of a solvent - 3°C" and the case of the highest internal liquid temperature ≤ "the melting point of a solvent - 3°C"). As a result, in the case of the highest internal liquid temperature > "the melting point of a solvent - 3°C" (C, G, and I), 80% or more of the pores were columnar/planar pores, and 20% or less of the pores were spherical pores. On the other hand, in the case of the highest internal liquid temperature ≤ "the melting point of a solvent - 3°C" (A, B, D, E, F, and H), 50% or more of the pores were spherical pores. In addition, in the porous bodies regarding which the highest internal liquid temperature ≤ "the melting point of a solvent - 7°C" (A, B, and D), 80% or more of the pores were spherical pores, and thus, almost the entire porous body was constituted with spherical pores. From these results, it was found that, in order to prepare a porous body, pores of which have a spherical shape, it is important to set the highest internal liquid temperature in an unfrozen state at "the melting point of a solvent - 3°C" or lower, and that when "the melting point of a solvent that is set at -7°C" or lower, almost all pores became spherical pores.

The pore shapes and mean pore sizes obtained under the aforementioned conditions A to I are as follows.

A: Spherical pores: 100%, 62.74 μm
B: Spherical pores: 100%, 65.36 μm
C: Columnar/planar pores: 90%, 79.19 μm
D: Spherical pores: 100%, 63.17 μm
E: Spherical pores: 70%, 69.44 μm
F: Spherical pores: 50%, 53.98 μm
G: Columnar/planar pores: 90%, 79.48 μm
H: Spherical pores: 80%, 76.58 μm
I: Columnar/planar pores: 90%, 79.65 μm

[Example 22] Production of recombinant peptide blocks (crushing and crosslinking of porous bodies)

The CBE3 porous bodies obtained in Example 21 were crushed using New Power Mill (Osaka Chemical Co.., Ltd., New Power Mill PM-2005). Crushing was carried out at a maximum engine speed for 1 minute × 5 times, namely, for 5 minutes in total. Using a sieve made of stainless steel, the crushed product was divided in terms of size, so as to obtain recombinant peptide blocks with sizes of 25 to 53 μm and 53 to 106 μm. Thereafter, the blocks were subjected to thermal crosslinking at 160°C under reduced pressure for 72 hours, thereby obtaining samples. These samples satisfied the conditions that the tap density is 10 mg/cm$^3$ or more and 500 mg/cm$^3$ or less, or that the value of the square root of the cross-sectional area / boundary length of the polymer block in a two-dimensional sectional image is 0.01 or more and 0.13 or less. All of the mosaic cell masses produced in the same manners as those of Example 10 and Example 12 using these samples exhibited higher performance results (high survival rate of cells) than comparative blocks in terms of the same *in vitro* assay as that in Example 14 and the results of transplantation in animals according to the same evaluations as those in Examples 16 to 18, regardless of A to I. However, there was a small difference in terms of performance among these A to I, and specifically, A, B and D had the highest performance, and then, the performance of E, F, and H was high after A, B and D, and the performance of C, G and I came after E, F and H. That is to say, these results demonstrate that a difference in performance may be generated, depending on the shape of pores comprised in a porous body. In Example 18, D (corresponding to "B" in Example 2) is described in detail as typical results.

## Claims

1. A cell construct for cell transplantation comprising biocompatible polymer blocks that do not contain glutaraldehyde and at least one type of cells, wherein a plurality of biocompatible polymer blocks are disposed in gaps among a plurality of cells, and wherein the biocompatible polymer blocks have a tap density of 10 $mg/cm^3$ or more and 500 $mg/cm^3$ or less, or the value of the square root of the cross-sectional area / boundary length in the two-dimensional sectional image of the polymer block is 0.01 or more and 0.13 or less, wherein the size of one biocompatible polymer block is 20 μm or more and 200 μm or less.

2. The cell construct for cell transplantation according to claim 1, wherein biocompatible polymers are crosslinked by heat, an ultraviolet ray or an enzyme in the biocompatible polymer block, and the biocompatible polymer block has a degree of cross-linkage of 6 or more, and also has a water absorption percentage of 300% or more.

3. The cell construct for cell transplantation according to any one of claims 1 to 2, wherein the biocompatible polymer block is obtained by crushing the porous body of a biocompatible polymer, and the porous body of the biocompatible polymer has the following properties (a) and (b):

   (a) it has a porosity of 81% or more and 99.99% or less; and
   (b) pores with a size of 20 to 200 μm have a space occupation percentage of 85% or more.

4. The cell construct for cell transplantation according to any one of claims 1 to 3, which has a thickness or a diameter of 400 μm or more and 3 cm or less.

5. The cell construct for cell transplantation according to any one of claims 1 to 4, wherein the biocompatible polymer is recombinant gelatin.

6. The cell construct for cell transplantation according to claim 5, wherein the recombinant gelatin has (1) the amino acid sequence shown in SEQ ID NO: 1 or (2) an amino acid sequence showing homology of 80% or more with the amino acid sequence shown in SEQ ID NO: 1 and having biocompatibility.

7. The cell construct for cell transplantation according to any one of claims 1 to 6, wherein the cells comprise both non-vascular cells and vascular cells.

8. A biocompatible polymer block that does not contain glutaraldehyde, wherein the biocompatible polymer block has a tap density of 10 $mg/cm^3$ or more and 500 $mg/cm^3$ or less, or the value of the square root of the cross-sectional area / boundary length in the two-dimensional sectional image of the polymer block is 0.01 or more and 0.13 or less, wherein the size of the biocompatible polymer block is 20 μm or more and 200 μm or less.

9. The biocompatible polymer block according to claim 8, wherein biocompatible polymers are crosslinked by heat, an ultraviolet ray or an enzyme, and which has a degree of cross-linkage of 6 or more, and also has a water absorption percentage of 300% or more.

10. The biocompatible polymer block according to claim 8 or 9, which is obtained by crushing the porous body of a biocompatible polymer, wherein the porous body of the biocompatible polymer has the following properties (a) and (b):

    (a) it has a porosity of 81% or more and 99.99% or less; and
    (b) pores with a size of 20 to 200 μm have a space occupation percentage of 85% or more.

11. The biocompatible polymer block according to claim 9 or 10, wherein the biocompatible polymer is recombinant gelatin.

12. The biocompatible polymer block according to claim 10 or 11, wherein the recombinant gelatin has (1) the amino acid sequence shown in SEQ ID NO: 1 or (2) an amino acid sequence showing homology of 80% or more with the amino acid sequence shown in SEQ ID NO: 1 and having biocompatibility.

**13.** A method for producing the cell construct for cell transplantation according to any one of claims 1 to 7, which comprises mixing the biocompatible polymer blocks according to any one of claims 8 to 12 with at least one type of cells.

**14.** A method for producing the porous body of a biocompatible polymer block of any one of claims 8-12, which comprises:

(a) a step of freezing a solution of the biocompatible polymer by a freezing treatment, in which the liquid temperature of the portion having the highest liquid temperature in the solution (highest internal liquid temperature) becomes "the melting point of a solvent - 3°C" or lower in an unfrozen state; and
(b) a step of freeze-drying the frozen biocompatible polymer obtained in the step (a).

**Patentansprüche**

**1.** Zellkonstrukt für Zelltransplantation umfassend biokompatible Polymer-Blöcke, die kein Glutaraldehyd und mindestens eine Zellart enthalten, wobei eine Pluralität von biokompatiblen Polymer-Blöcken in Lücken zwischen einer Pluralität von Zellen disponiert sind, und wobei die biokompatiblen Polymer-Blöcke eine Klopfdichte von 10 mg/cm$^3$ oder mehr und 500 mg/cm$^3$ oder weniger haben, oder der Wert der Quadratwurzel der Querschnittsfläche / Abgrenzungslänge in dem zweidimensionalen Schnittbild des Polymer-Blocks 0,01 oder mehr und 0,13 oder weniger ist, wobei die Größe von einem biokompatiblen Polymer-Block 20 μm oder mehr und 200 μm oder weniger ist.

**2.** Zellkonstrukt für Zelltransplantation gemäß Anspruch 1, wobei biokompatible Polymere durch Hitze, einem ultravioletten Lichtstrahl oder ein Enzym in dem biokompatiblen Polymer-Block vernetzt werden, und der biokompatible Polymer-Block einen Grad der Vernetzung von 6 oder mehr hat, und auch eine prozentuale Wasserabsorption von 300% oder mehr hat.

**3.** Zellkonstrukt für Zelltransplantation gemäß einem der Ansprüche 1 oder 2, wobei der biokompatible Polymer-Block durch Zerdrücken des porösen Körpers eines biokompatiblen Polymers erhalten wird, und der poröse Körper des biokompatiblen Polymers die folgenden Eigenschaften (a) und (b) hat:

(a) er hat eine Porosität von 81% oder mehr und 99,99% oder weniger; und
(b) Poren mit einer Größe von 20 bis 200 μm haben eine prozentuale Raumnutzung von 85% oder mehr.

**4.** Zellkonstrukt für Zelltransplantation gemäß einem der Ansprüche 1 bis 3, welches eine Dicke oder einen Durchmesser von 400 μm oder mehr und 3 cm oder weniger hat.

**5.** Zellkonstrukt für Zelltransplantation gemäß einem der Ansprüche 1 bis 4, wobei das biokompatible Polymer rekombinante Gelatine ist.

**6.** Zellkonstrukt für Zelltransplantation gemäß Anspruch 5, wobei die rekombinante Gelatine (1) die Aminosäuresequenz dargestellt in SEQ ID NO: 1 hat, oder (2) eine Aminosäuresequenz hat, die Homologie von 80% oder mehr mit der Aminosäuresequenz dargestellt in SEQ ID NO: 1 aufweist und Biokompatibilität hat.

**7.** Zellkonstrukt für Zelltransplantation gemäß einem der Ansprüche 1 bis 6, wobei die Zellen sowohl nichtvaskuläre als auch vaskuläre Zellen umfassen.

**8.** Biokompatibler Polymer-Block, der kein Glutaraldehyd enthält, wobei der biokompatible Polymer-Block eine Klopfdichte von 10 mg/cm$^3$ oder mehr und 500 mg/cm$^3$ oder weniger hat, oder der Wert der Quadratwurzel der Querschnittsfläche / Abgrenzungslänge in dem zweidimensionalen Schnittbild des Polymer-Blocks 0,01 oder mehr und 0,13 oder weniger ist, wobei die Größe des biokompatiblen Polymer-Blocks 20 μm oder mehr und 200 μm oder weniger ist.

**9.** Biokompatibler Polymer-Block gemäß Anspruch 8, wobei biokompatible Polymere durch Hitze, einem ultravioletten Lichtstrahl oder ein Enzym vernetzt werden, und der einen Grad der Vernetzung von 6 oder mehr hat, und auch eine prozentuale Wasserabsorption von 300% oder mehr hat.

10. Biokompatibler Polymer-Block gemäß Anspruch 8 oder 9, welcher durch Zerdrücken des porösen Körpers eines biokompatiblen Polymers erhalten wird, wobei der poröse Körper des biokompatiblen Polymers die folgenden Eigenschaften (a) und (b) hat:

(a) er hat eine Porosität von 81% oder mehr und 99,99% oder weniger; und
(b) Poren mit einer Größe von 20 bis 200 μm haben eine prozentuale Raumnutzung von 85% oder mehr.

11. Biokompatibler Polymer-Block gemäß Anspruch 9 oder 10, wobei das biokompatible Polymer rekombinante Gelatine ist.

12. Biokompatibler Polymer-Block gemäß Anspruch 10 oder 11, wobei die rekombinante Gelatine (1) die Aminosäuresequenz dargestellt in SEQ ID NO: 1 hat, oder (2) eine Aminosäuresequenz hat, die Homologie von 80% oder mehr mit der Aminosäuresequenz dargestellt in SEQ ID NO: 1 aufweist und Biokompatibilität hat.

13. Verfahren zum Herstellen des Zellkonstrukts für Zelltransplantation gemäß einem der Ansprüche 1 bis 7, welches Mischen der biokompatiblen Polymer-Blöcke gemäß einem der Ansprüche 8 bis 12 mit mindestens einer Zellart umfasst.

14. Verfahren zum Herstellen des porösen Körpers eines biokompatiblen Polymer-Blocks gemäß einem der Ansprüche 8-12, welches umfasst:

(a) einen Einfrierschritt einer Lösung des biokompatiblen Polymers durch eine Gefrierbehandlung, worin die Flüssigkeitstemperatur des Teils mit der höchsten Flüssigkeitstemperatur in der Lösung (höchste interne Flüssigkeitstemperatur) "der Schmelzpunkt eines Lösungsmittels - 3°C" oder niedriger in einem aufgetauten Zustand wird; und
(b) einen Gefriertrocknungsschritt des in Schritt (a) erhaltenen gefrorenen biokompatiblen Polymers.

## Revendications

1. Construction cellulaire pour une transplantation cellulaire comprenant des blocs de polymère biocompatible qui ne contient pas de glutaraldéhyde et au moins un type de cellules, dans laquelle une pluralité de blocs de polymère biocompatible sont disposés dans des espaces parmi une pluralité de cellules, et dans laquelle les blocs de polymère biocompatible ont une masse volumique tassée de 10 $mg/cm^3$ ou plus et de 500 $mg/cm^3$ ou moins, ou la valeur de la racine carrée de la section transversale/longueur limite dans l'image en coupe bidimensionnelle du bloc de polymère est de 0,01 ou plus et de 0,13 ou moins, dans laquelle la taille d'un bloc de polymère biocompatible est de 20 μm ou plus et de 200 μm ou moins.

2. Construction cellulaire pour une transplantation cellulaire selon la revendication 1, dans laquelle des polymères biocompatibles sont réticulés par la chaleur, un rayon ultraviolet ou une enzyme dans le bloc de polymère biocompatible, et le bloc de polymère biocompatible a un degré de réticulation de 6 ou plus, et a également un pourcentage d'absorption d'eau de 300 % ou plus.

3. Construction cellulaire pour une transplantation cellulaire selon l'une quelconque des revendications 1 à 2, dans laquelle le bloc de polymère biocompatible est obtenu en écrasant le corps poreux d'un polymère biocompatible, et le corps poreux du polymère biocompatible a les propriétés (a) et (b) suivantes :

(a) il a une porosité de 81 % ou plus et de 99,99 % ou moins ; et
(b) des pores d'une taille de 20 à 200 μm ont un pourcentage d'occupation d'espace de 85 % ou plus.

4. Construction cellulaire pour une transplantation cellulaire selon l'une quelconque des revendications 1 à 3, qui a une épaisseur ou un diamètre de 400 μm ou plus et de 3 cm ou moins.

5. Construction cellulaire pour une transplantation cellulaire selon l'une quelconque des revendications 1 à 4, dans laquelle le polymère biocompatible est de la gélatine recombinante.

6. Construction cellulaire pour une transplantation cellulaire selon la revendication 5, dans laquelle la gélatine recombinante a (1) la séquence d'acides aminés indiquée dans SEQ ID NO : 1 ou (2) une séquence d'acides aminés présentant une homologie de 80% ou plus avec la séquence d'acides aminés indiquée dans SEQ ID NO : 1 et ayant une biocompatibilité.

**7.** Construction cellulaire pour une transplantation cellulaire selon l'une quelconque des revendications 1 à 6, dans laquelle les cellules comprennent à la fois des cellules non vasculaires et des cellules vasculaires.

**8.** Bloc de polymère biocompatible ne contenant pas de glutaraldéhyde, dans lequel le bloc de polymère biocompatible a une masse volumique tassée de 10 mg/cm$^3$ ou plus et de 500 mg/cm$^3$ ou moins, ou la valeur de la racine carrée de la section transversale/longueur limite dans l'image en coupe bidimensionnelle du bloc de polymère est de 0,01 ou plus et de 0,13 ou moins, dans lequel la taille du bloc de polymère biocompatible est de 20 μm ou plus et de 200 μm ou moins.

**9.** Bloc de polymère biocompatible selon la revendication 8, dans lequel des polymères biocompatibles sont réticulés par la chaleur, un rayon ultraviolet ou une enzyme, et qui a un degré de réticulation de 6 ou plus, et qui a également un pourcentage d'absorption d'eau de 300 % ou plus.

**10.** Bloc de polymère biocompatible selon la revendication 8 ou 9, qui est obtenu en écrasant le corps poreux d'un polymère biocompatible, dans lequel le corps poreux du polymère biocompatible a les propriétés (a) et (b) suivantes :

(a) il a une porosité de 81 % ou plus et de 99,99 % ou moins ; et
(b) des pores d'une taille de 20 à 200 μm ont un pourcentage d'occupation d'espace de 85 % ou plus.

**11.** Bloc de polymère biocompatible selon la revendication 9 ou 10, dans lequel le polymère biocompatible est de la gélatine recombinante.

**12.** Bloc de polymère biocompatible selon la revendication 10 ou 11, dans laquelle la gélatine recombinante a (1) la séquence d'acides aminés indiquée dans SEQ ID NO : 1 ou (2) une séquence d'acides aminés présentant une homologie de 80 % ou plus avec la séquence d'acides aminés indiquée dans SEQ ID NO : 1 et ayant une biocompatibilité.

**13.** Procédé pour produire la construction cellulaire pour une transplantation cellulaire selon l'une des revendications 1 à 7, qui comprend le mélange des blocs de polymère biocompatible selon l'une quelconque des revendications 8 à 12 avec au moins un type de cellules.

**14.** Procédé de production du corps poreux d'un bloc de polymère biocompatible selon l'une des revendications 8 à 12, qui comprend :

(a) une étape de congélation d'une solution du polymère biocompatible par un traitement de congélation, où la température de liquide de la partie ayant la température de liquide la plus élevée dans la solution (température de liquide interne la plus élevée) devient « le point de fusion d'un solvant -3 °C » ou moins à l'état non congelé ; et
(b) une étape de lyophilisation du polymère biocompatible congelé obtenu à l'étape (a).

[Figure 1]

Figure 1. Difference among blocks in *in vitro* ATP assay

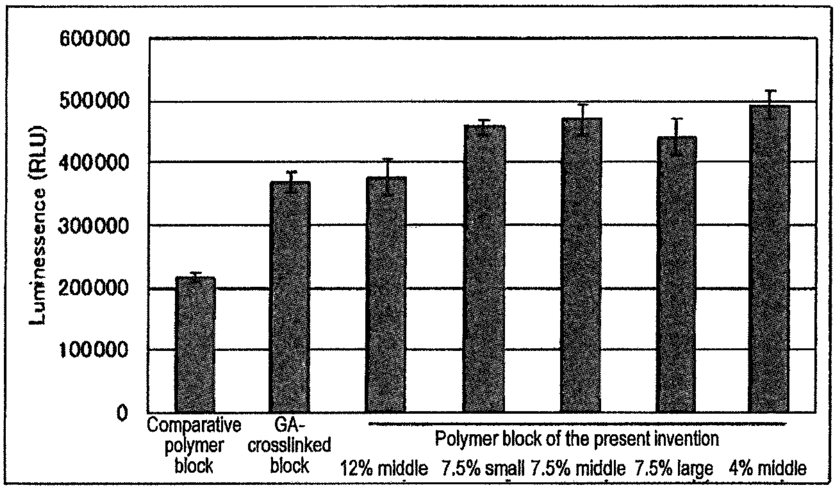

[Figure 2]

Figure 2. Difference in survival states of cells in hMSC mosaic cell masses comprising various types of blocks (2 weeks later)

A
GA-crosslinked μ block

B
Comparative block

C
Block of the present invention

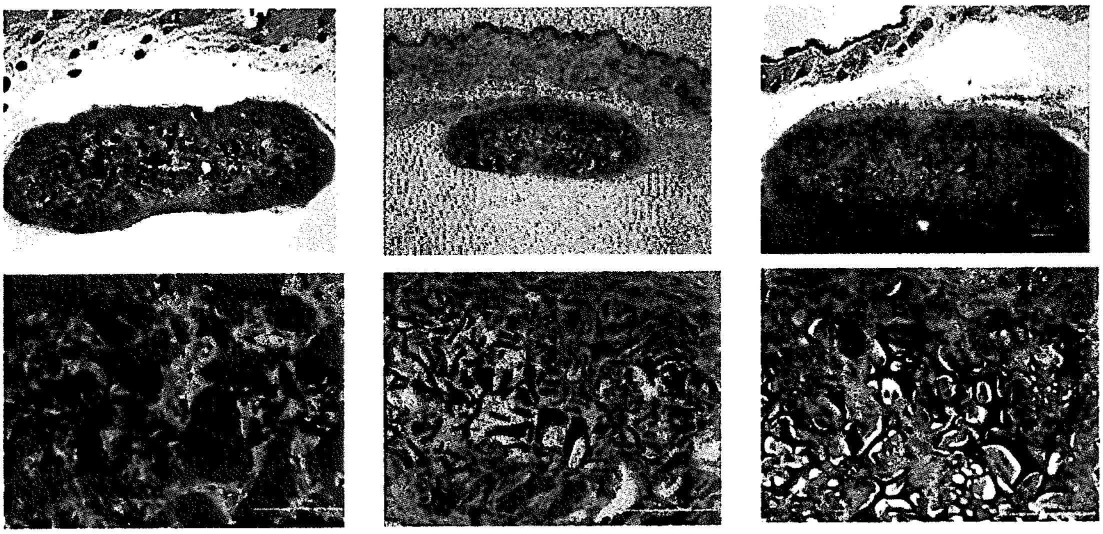

A: Large quantities of survival cells (100 cells)
Survival rate: 80%

B: Small quantities of survival cells (37 cells)
Survival rate: 45%

C: Large quantities of survival cells (116 cells)
Survival rate: 84%

[Figure 3]

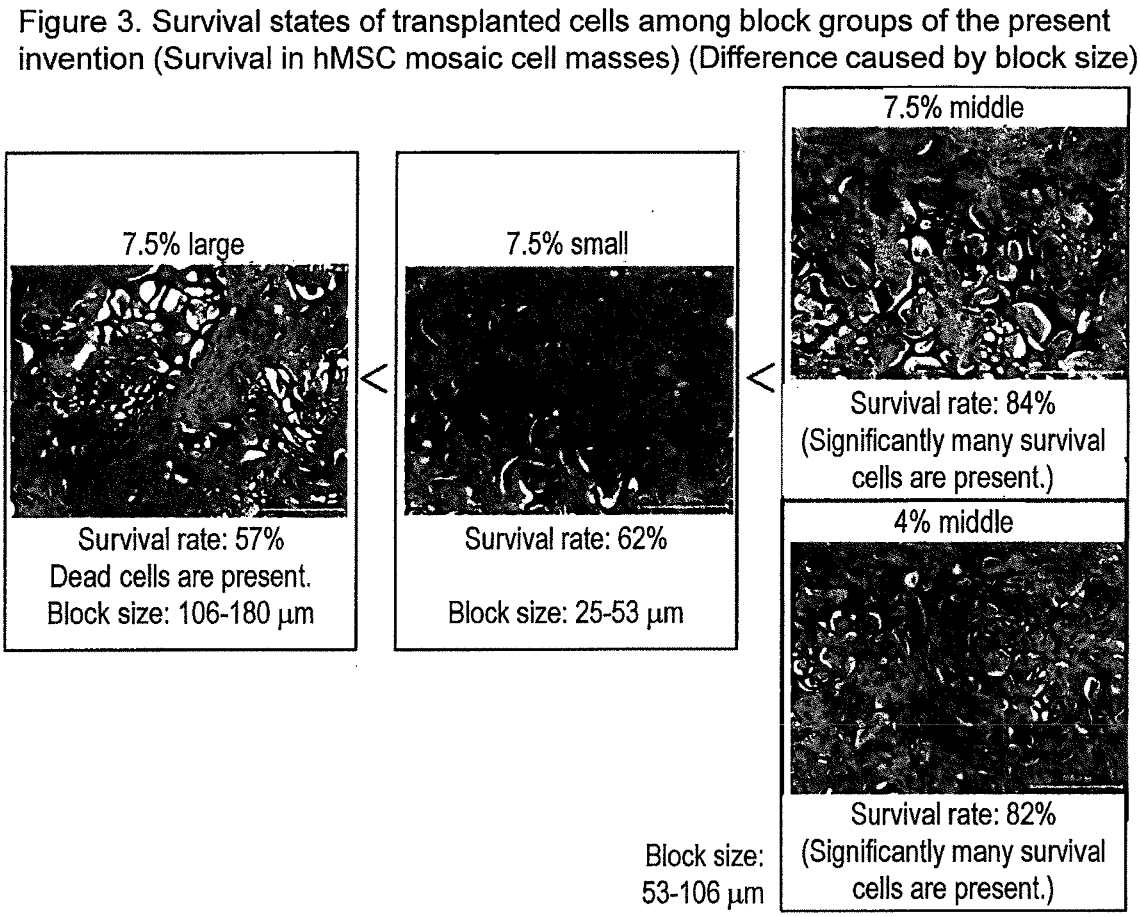
Figure 3. Survival states of transplanted cells among block groups of the present invention (Survival in hMSC mosaic cell masses) (Difference caused by block size)
7.5% large
Survival rate: 57%
Dead cells are present.
Block size: 106-180 µm
<
7.5% small
Survival rate: 62%
Block size: 25-53 µm
<
7.5% middle
Survival rate: 84%
(Significantly many survival cells are present.)
4% middle
Survival rate: 82%
(Significantly many survival cells are present.)
Block size: 53-106 µm

[Figure 4]

Figure 4. Angiogenesis in hMSC mosaic cell mass 2 weeks after transplantation

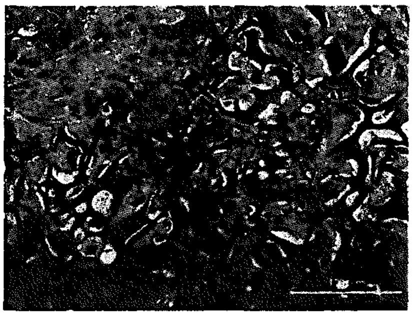

Blood vessels are formed.
63 blood vessels/$mm^2$

[Figure 5]

Figure 5. Angiogenesis in hMSC + hECFC mosaic cell mass 2 weeks after transplantation

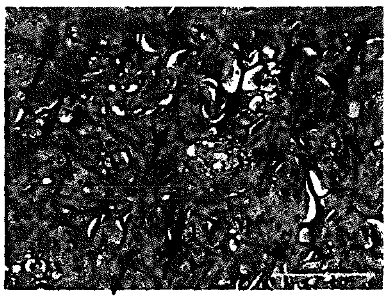

Many blood vessels are formed.
180 blood vessels/$mm^2$

[Figure 6]

Figure 6. Space occupation percentages of individual pore sizes in porous bodies

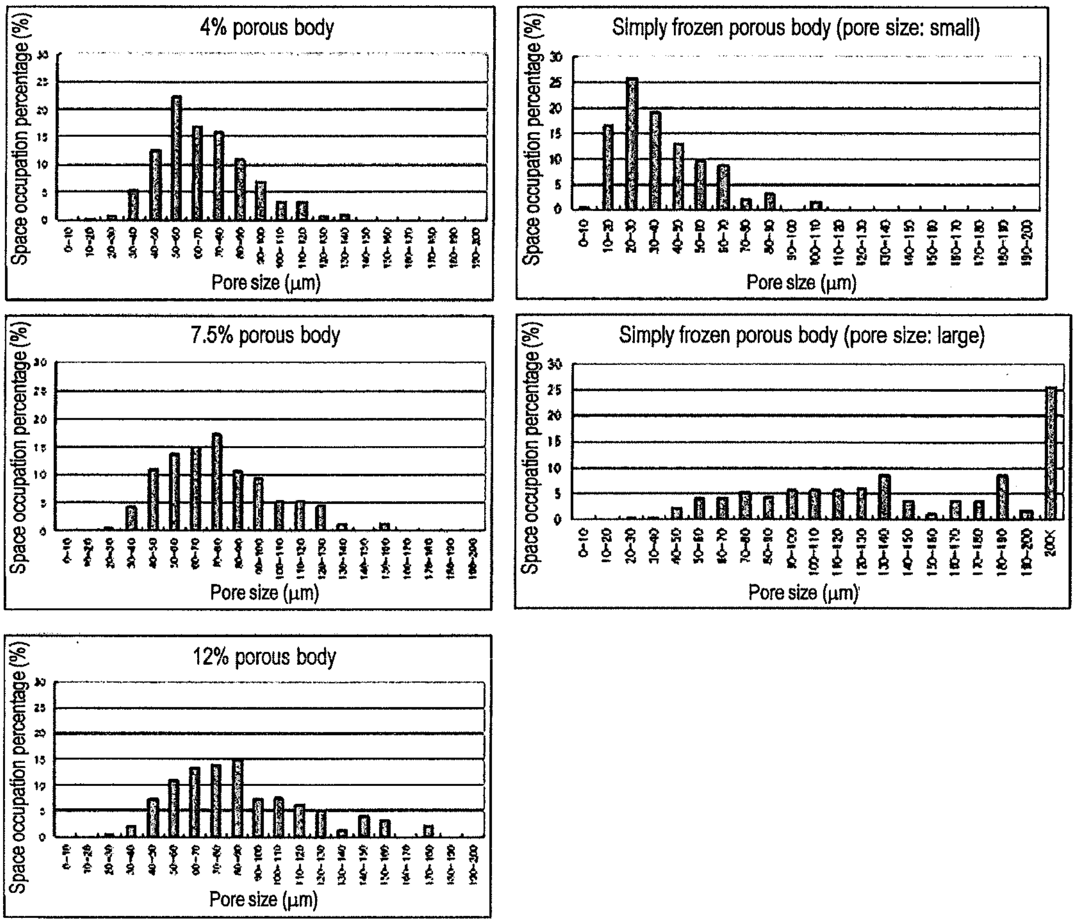

[Figure 7]

Figure 7. HE section images of CBE3 porous bodies, and pore shapes and highest internal liquid temperatures

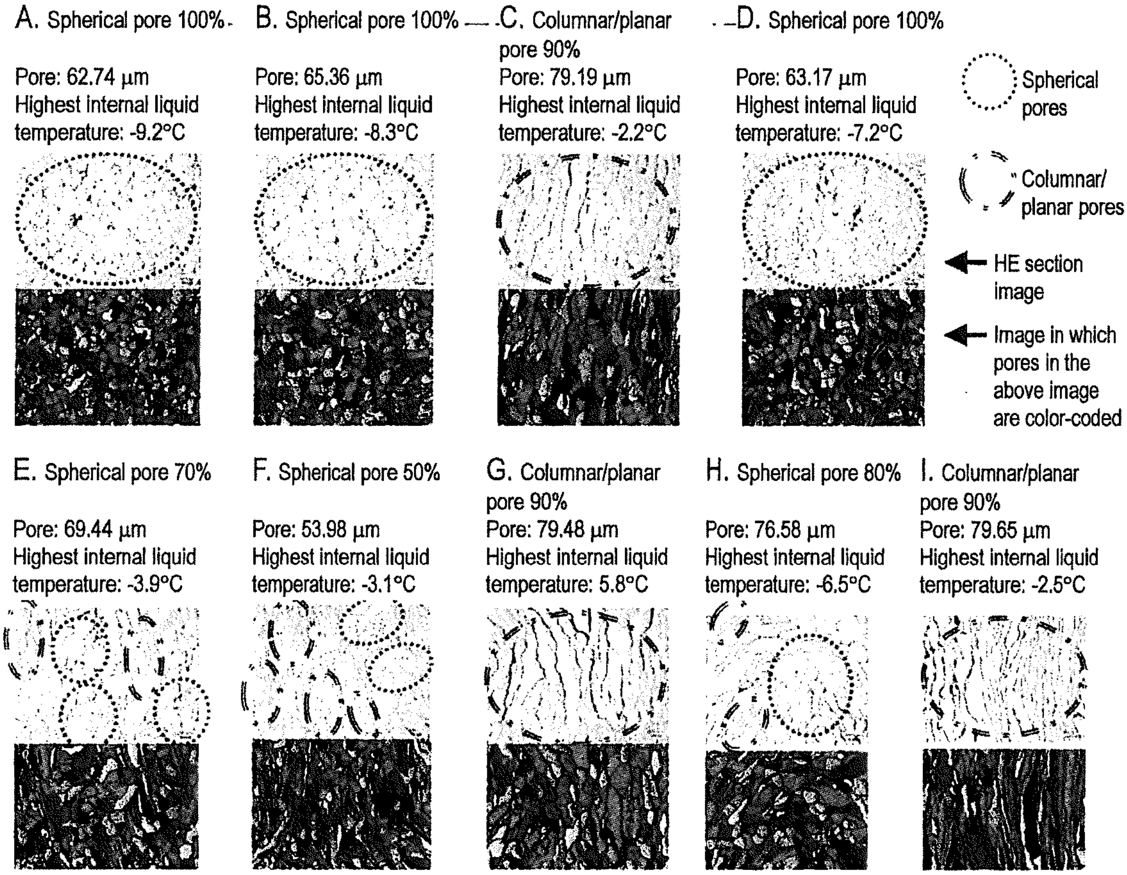

[Figure 8]

Figure 8. Change over time in highest internal liquid temperature at A. shelf board temperature of -40°C (glass board: 2.2 mm)

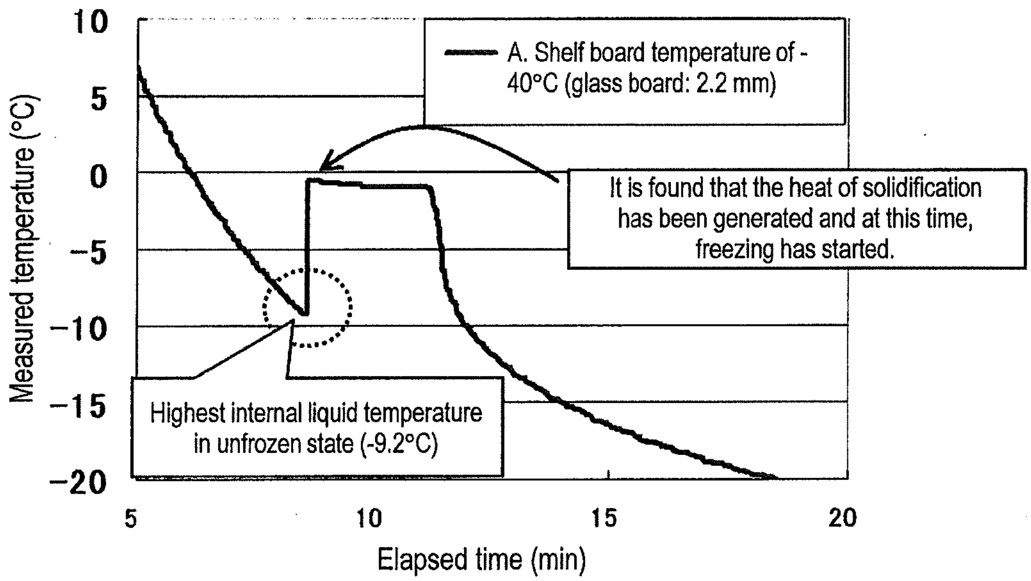

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2543397 A
- WO 2011108517 A
- EP 1014176 A
- US 6992172 B
- WO 200485473 A
- WO 2008103041 A
- EP 1014176 A2


### Non-patent literature cited in the description


- **SHIMIZU, T. et al.** *Circ. Res.,* 2002, vol. 90, e40-48
- **KUSHIDA, A. et al.** *J. Biomed. Mater. Res.,* 2000, vol. 51, 216-223
- **KUSHIDA, A. et al.** *J. Biomed. Mater. Res.,* 1999, vol. 45, 355-362
- **SHIMIZU, T. ; YAMATO, M. ; KIKUCHI, A. ; OKANO, T.** *Tissue Eng.,* 2001, vol. 7, 141-151
- **SHIMIZU, T et al.** *J. Biomed. Mater. Res.,* 2002, vol. 60, 110-117
- **HARIMOTO, M. et al.** *J. Biomed. Mater. Res.,* 2002, vol. 62, 464-470
- **SHIMIZU, T. ; YAMATO, M. ; KIKUCHI, A. ; OKANO, T.** *Biomaterials,* 2003, vol. 24, 2309-2316
- *Inflammation and Regeneration,* 2005, vol. 25 (3), 158-159
- Toward Fusion of Study of Inflammation with Regenerative Medicine. the 26th Annual Meeting of the Japanese Society of Inflammation and Regeneration
- *Pharmaceutical Bulletin,* 1954, vol. 2 (2), 163-173
- *Journal of Japanese Chemistry,* 1957, vol. 11 (10), 719-725
- *Fragrance Journal,* 1981, vol. 50, 79-82
- Shinban Yuuki Gainenzu -Kiso to Ouyou. **YOSHIO KODA et al.** The Organic Conceptual Diagram, its Fundamentals and Applications in English. Sankyo Publishing Co., Ltd, 2008
- Protein Identification and Analysis Tools on the ExPASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788
- *Medicina Philosophica,* 1990, vol. 9 (7), 527

SEQUENCE LISTING

<110> FUJIFILM Corporation
<120> A cell structure for cell transplantation, a biocompatible polymer block and a method for preparation thereof
<130>\201@13F03654
<160> 1
<170> PatentIn version 3.5

<210> 1
<211> 571
<212> PRT
<213> Recombinant
<400> 1

Gly Ala Pro Gly Ala Pro Gly Leu Gln Gly Ala Pro Gly Leu Gln Gly
1 5 10 15
Met Pro Gly Glu Arg Gly Ala Ala Gly Leu Pro Gly Pro Lys Gly Glu
20 25 30
Arg Gly Asp Ala Gly Pro Lys Gly Ala Asp Gly Ala Pro Gly Ala Pro
35 40 45
Gly Leu Gln Gly Met Pro Gly Glu Arg Gly Ala Ala Gly Leu Pro Gly
50 55 60
Pro Lys Gly Glu Arg Gly Asp Ala Gly Pro Lys Gly Ala Asp Gly Ala
65 70 75 80
Pro Gly Lys Asp Gly Val Arg Gly Leu Ala Gly Pro Ile Gly Pro Pro
85 90 95
Gly Glu Arg Gly Ala Ala Gly Leu Pro Gly Pro Lys Gly Glu Arg Gly
100 105 110
Asp Ala Gly Pro Lys Gly Ala Asp Gly Ala Pro Gly Lys Asp Gly Val
115 120 125
Arg Gly Leu Ala Gly Pro Ile Gly Pro Pro Gly Pro Ala Gly Ala Pro
130 135 140
Gly Ala Pro Gly Leu Gln Gly Met Pro Gly Glu Arg Gly Ala Ala Gly
145 150 155 160
Leu Pro Gly Pro Lys Gly Glu Arg Gly Asp Ala Gly Pro Lys Gly Ala
165 170 175
Asp Gly Ala Pro Gly Lys Asp Gly Val Arg Gly Leu Ala Gly Pro Pro
180 185 190
Gly Ala Pro Gly Leu Gln Gly Ala Pro Gly Leu Gln Gly Met Pro Gly
195 200 205
Glu Arg Gly Ala Ala Gly Leu Pro Gly Pro Lys Gly Glu Arg Gly Asp
210 215 220
Ala Gly Pro Lys Gly Ala Asp Gly Ala Pro Gly Ala Pro Gly Leu Gln
225 230 235 240
Gly Met Pro Gly Glu Arg Gly Ala Ala Gly Leu Pro Gly Pro Lys Gly
245 250 255
Glu Arg Gly Asp Ala Gly Pro Lys Gly Ala Asp Gly Ala Pro Gly Lys
260 265 270
Asp Gly Val Arg Gly Leu Ala Gly Pro Ile Gly Pro Pro Gly Glu Arg
275 280 285
Gly Ala Ala Gly Leu Pro Gly Pro Lys Gly Glu Arg Gly Asp Ala Gly
290 295 300
Pro Lys Gly Ala Asp Gly Ala Pro Gly Lys Asp Gly Val Arg Gly Leu
305 310 315 320
Ala Gly Pro Ile Gly Pro Pro Gly Pro Ala Gly Ala Pro Gly Ala Pro
325 330 335
Gly Leu Gln Gly Met Pro Gly Glu Arg Gly Ala Ala Gly Leu Pro Gly
340 345 350
Pro Lys Gly Glu Arg Gly Asp Ala Gly Pro Lys Gly Ala Asp Gly Ala
355 360 365
Pro Gly Lys Asp Gly Val Arg Gly Leu Ala Gly Pro Pro Gly Ala Pro
370 375 380
Gly Leu Gln Gly Ala Pro Gly Leu Gln Gly Met Pro Gly Glu Arg Gly
385 390 395 400
Ala Ala Gly Leu Pro Gly Pro Lys Gly Glu Arg Gly Asp Ala Gly Pro

```
                    405                 410                 415
Lys Gly Ala Asp Gly Ala Pro Gly Ala Pro Gly Leu Gln Gly Met Pro
                420                 425                 430
Gly Glu Arg Gly Ala Ala Gly Leu Pro Gly Pro Lys Gly Glu Arg Gly
            435                 440                 445
Asp Ala Gly Pro Lys Gly Ala Asp Gly Ala Pro Gly Lys Asp Gly Val
    450                 455                 460
Arg Gly Leu Ala Gly Pro Ile Gly Pro Pro Gly Glu Arg Gly Ala Ala
465                 470                 475                 480
Gly Leu Pro Gly Pro Lys Gly Glu Arg Gly Asp Ala Gly Pro Lys Gly
                    485                 490                 495
Ala Asp Gly Ala Pro Gly Lys Asp Gly Val Arg Gly Leu Ala Gly Pro
                500                 505                 510
Ile Gly Pro Pro Gly Pro Ala Gly Ala Pro Gly Ala Pro Gly Leu Gln
            515                 520                 525
Gly Met Pro Gly Glu Arg Gly Ala Ala Gly Leu Pro Gly Pro Lys Gly
    530                 535                 540
Glu Arg Gly Asp Ala Gly Pro Lys Gly Ala Asp Gly Ala Pro Gly Lys
545                 550                 555                 560
Asp Gly Val Arg Gly Leu Ala Gly Pro Pro Gly
                    565                 570
```

<210> 2
<211> 4
<212> PRT
<213> Artificial Sequence
<220>
<223> Description of Artificial Sequence: adhesive sequence
<400> 2

```
Arg Glu Asp Val
1
```

<210> 3
<211> 5
<212> PRT
<213> Artificial Sequence
<220>
<223> Description of Artificial Sequence: adhesive sequence
<400> 3

```
Tyr Ile Gly Ser Arg
1               5
```

<210> 4
<211> 5
<212> PRT
<213> Artificial Sequence
<220>
<223> Description of Artificial Sequence: adhesive sequence
<400> 4

```
Pro Asp Ser Gly Arg
1               5
```

<210> 5
<211> 7
<212> PRT

<213> Artificial Sequence
<220>
<223> Description of Artificial Sequence: adhesive sequence
<400> 5

```
Arg Tyr Val Val Leu Pro Arg
1               5
```

<210> 6
<211> 6
<212> PRT
<213> Artificial Sequence
<220>
<223> Description of Artificial Sequence: adhesive sequence
<400> 6

```
Leu Gly Thr Ile Pro Gly
1               5
```

<210> 7
<211> 10
<212> PRT
<213> Artificial Sequence
<220>
<223> Description of Artificial Sequence: adhesive sequence
<400> 7

```
Arg Asn Ile Ala Glu Ile Ile Lys Asp Ile
1               5                   10
```

<210> 8
<211> 5
<212> PRT
<213> Artificial Sequence
<220>
<223> Description of Artificial Sequence: adhesive sequence
<400> 8

```
Ile Lys Val Ala Val
1               5
```

<210> 9
<211> 4
<212> PRT
<213> Artificial Sequence
<220>
<223> Description of Artificial Sequence: adhesive sequence
<400> 9

```
Asp Gly Glu Ala
1
```

<210> 10
<211> 4
<212> PRT
<213> Artificial Sequence
<220>
<223> Description of Artificial Sequence: adhesive sequence

<400> 10

Glu Arg Gly Asp
1